(19) **Europäisches Patentamt European Patent Office Office européen des brevets**

(11) **EP 2 531 527 B1**

(12) **EUROPEAN PATENT SPECIFICATION**

(45) Date of publication and mention
of the grant of the patent:
**19.03.2014 Bulletin 2014/12**

(51) Int Cl.:
*C07K 16/28* (2006.01)        *A61K 39/395* (2006.01)
*A61P 35/00* (2006.01)

(21) Application number: **11703182.3**

(22) Date of filing: **02.02.2011**

(86) International application number:
**PCT/EP2011/051436**

(87) International publication number:
**WO 2011/095498 (11.08.2011 Gazette 2011/32)**

(54) **A MONOCLONAL ANTIBODY TO CD44 FOR USE IN THE TREATMENT OF HEAD AND NECK SQUAMOUS CELL CARCINOMA**

MONOKLONALER ANTIKÖRPER GEGEN CD44 ZUR VERWENDUNG BEI DER BEHANDLUNG VON KOPF-HALS-PLATTENEPITHELKARZINOM

ANTICORPS MONOCLONAL ANTI-CD44 DESTINÉ À ÊTRE UTILISÉ POUR TRAITER LE CARCINOME À CELLULES SQUAMEUSES DE LA TÊTE ET DU COU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.02.2010 US 301449 P**

(43) Date of publication of application:
**12.12.2012 Bulletin 2012/50**

(73) Proprietors:
• **F.Hoffmann-La Roche AG**
  **4070 Basel (CH)**
• **University of Miami**
  **Miami, FL 33136 (US)**

(72) Inventors:
• **DA CRUZ, Luis A.G.**
  **Toronto**
  **Ontario M2M 2V9 (CA)**

• **FRANZMANN, Elizabeth, Jane**
  **Miami**
  **Florida 33156 (US)**

(74) Representative: **Vossius & Partner**
  **Siebertstrasse 4**
  **81675 München (DE)**

(56) References cited:
**US-A1- 2003 103 985    US-A1- 2009 004 103**

• **L. DA CRUZ ET AL.: "ARH460-16-2, targeting the CD44 cancer stem cell marker, uses multiple mechanisms to achieve its therapeutic anti-cancer effects.", EUROPEAN JOURNAL OF CANCER. SUPPLEMENT, vol. 6, no. 12, 1 October 2008 (2008-10-01), page 170, XP025534602, Oxford, GB**

**Description**

The present invention relates to methods for preventing, treating or diagnosing head and neck squamous cell carcinoma (HNSCC) using one or more cancerous disease modifying antibodies (CDMABs). In certain aspects, the CDMAB is an antibody specific for CD44. Also disclosed are combination therapy methods, comprising the use of a CDMAB according to the invention, optionally combined with one or more second CDMABs and/or chemotherapeutic agents, as a means for preventing or treating cancer.

Head and neck squamous cell carcinoma (HNSCC) is a debilitating and deadly disease that usually presents in late stage and strikes 50,000 people in the United States and 500,000 individuals worldwide each year. HNSCC is a disease that causes significant morbidity, especially with respect to speech and swallowing functions. Surgery, radiation therapy, chemotherapy, or combinations of these are generally available as treatment options.

Despite rigorous combinations of surgery, chemotherapy and radiation, cure rates only reach 30% for late stage disease, and recurrence remains the most common cause of failure after standard therapies (in 40%-50% of patients). Salvage therapy consists of the same treatment options as for first line therapy. However, palliative surgery is often difficult and disfiguring. Furthermore, radiation therapy is rarely feasible or beneficial, and chemotherapy does not substantially improve survival rates in HNSCC patients. Prognosis for these patients remains poor, such that the median survival after recurrence is only approximately six months.

The poor outcomes may be due to ineffective killing of tumor initiating cells or cancer stem cells (CSCs). CSCs are capable of self renewal and also generate progeny with a differentiated phenotype and limited self-renewal. CSC, like normal stem cells, are resistant to chemotherapy and radiation therapy due to survival advantages such as increased DNA repair capacity. In fact, standard chemotherapy, e.g., cisplatin, and radiation therapy regimens actually expand the stem cell population.

CD44 plays a critical role in tumorigenesis. A transmembrane glycoprotein, CD44 binds hyaluronic acid (HA) in the extracellular matrix and cytoskeletal proteins. Interactions between CD44, HA, cytoskeletal components, and signaling molecules such as cyclin D1, EGFR, Rho and Ras promote growth and migration. Additionally, CD44 is released from the membrane into soluble form (solCD44) by matrix metalloproteinases (MMPs), an event that is critical for cell migration. CD44 has also been found to be an important CSC marker, found in breast cancer, colorectal cancers and HNSCC. CD44 activity has also been shown to exhibit activity in the formation of tumors in xenograft models; CD44+ HNSCC tumor cells are tumorigenic in immunocompromised mice, whereas control CD44-HNSCC tumor cells did produce tumors in the same models.

VFF-18, also designated as BIWA 1, is a high-affinity antibody to the v6 variant of CD44, specific for the 360-370 region of the polypeptide. Various versions or derivatives of BIWA 1 have undergone clinical testing for the treatment of cancer. In particular, BIWA 1 was been used as a $^{99m}$Technetium-labelled conjugate in a Phase 1 clinical trial for the testing of safety and targeting potential in 12 patients with squamous cell carcinoma of the head and neck. Forty hours after injection, 14 percent of the injected dose was taken up by the tumor, with minimal accumulation in other organs such as the kidney, spleen and bone marrow. Although the highly selective tumor binding suggests a role for BIWA 1 in radioimmunotherapy, the exceptionally high affinity of this antibody prevented penetration into the deeper layers of the tumor. Further, BIWA 1 was also found to be significantly immunogenic. Eleven of the twelve study patients developed human anti-mouse antibodies (HAMA) that exhibited heterogenous accumulation throughout the tumor and led to formation of antibody-soluble CD44 complexes.

WO 02/094879 discloses humanized versions of VFF-18 designed to overcome the HAMA response, designated BIWA 4 and BIWA 8. BIWA 4 was found to have a significantly lower antigen binding affinity compared to the parent VFF 18 antibody. However, the lower affinity BIWA 4 antibody demonstrated superior tumor uptake characteristics relative to the higher affinity BIWA 8. Both $^{99m}$Technetium-labelled and $^{186}$Rhenium-labelled BIWA 4 antibodies were assessed in a 33 patient Phase 1 clinical trial to determine safety, tolerability, tumor accumulation and, for $^{186}$Re-labelled BIWA 4, maximum tolerated dose. There appeared to be tumor related uptake of $^{99m}$Tc-labelled BIWA 4. There were no tumor responses observed at any dose of $^{186}$Re-labelled BIWA 4. Although a number of patients demonstrated stable disease throughout the course of the study; the dose limiting toxicity occurred at 60 mCi/m$^2$. There was also a 50-65 percent rate of adverse events with 12 of 33 patients deemed to have serious adverse events (thrombocytopenia, leucopenia and fever). Additionally, 6 of these 12 patients, all treated with $^{186}$Re-labelled BIWA 4, died in the course of treatment or follow-up due to disease progression. Two patients also developed human anti-human antibodies (HAHA). Accordingly, the radio-labeled VFF-18 failed to demonstrate any clinical effects.

A Phase 1 dose escalation trial of $^{186}$Re-labelled BIWA 4 was also performed in 20 patients. Oral mucositis and dose-limiting thrombocytopenia and leucocytopenia were observed; one patient developed a HAHA response. Stable disease was seen in 5 patients treated at the highest dose of 60 mCi/m$^2$. Although deemed to be acceptable in both safety and tolerability in this does range, the studies resulted only in stabilization of disease. Additionally, these studies have higher rates of adverse events compared to other non-radioisotope conjugated biological therapies in clinical studies.

U.S. Patent Application US 2003/0103985 discloses a humanized version of VFF-18 (BIWA 4) conjugated to a maytansinoid for use in tumor therapy, designated BIWI 1. BIWI 1 was found to have significant anti-tumor effects in mouse models of human epidermoid carcinoma of the vulva, squamous cell carcinoma of the pharynx and breast carcinoma. The unconjugated version, *i.e.*, BIWA 4, did not exhibit anti-tumor effects. The conjugate BIWI 1 has no evidence of safety or efficacy in humans.

Mab U36 is a murine monoclonal IgG1 antibody developed by immunization with UM-SCC-22B human hypopharyngeal carcinoma cells and was selected for cancer and tissue specificity. Antigen characterization through cDNA cloning and sequence analysis identified the epitope of Mab U36 to be within the v6 domain of keratinocyte-specific CD44 splice variant epican. Immunohistochemistry studies show the recognized epitope to be restricted to the cell membrane. Mab U36 labeled 94 percent of the head and neck squamous cell carcinomas (HNSCC) strongly, and within these tumors there was uniformity in cell staining.

Da Cruz, (2008), European J. of Cancer, Supplement, 6, page 170 mentions the expression of CD44 on cancer cells and that humanized anti-CD44 antibody ARH460-16-2 is in development for treatment. US 2009/004103 discloses chimeric ARH460-16-2 anti-CD44 inhibits tumor growth in human breast cancer or prostatic cancer cells in xenograft models.

A 10 patient $^{99m}$Tc-labelled Mab U36 study showed selective accumulation of the antibody to HNSCC cancers (20.4 +/- 12.4 percent injected dose/kg at 2 days); no adverse effects were reported but two patients developed HAMA. In a study of radio-iodinated murine Mab U36, there were 3 cases of HAMA in 18 patients and selective homogenous uptake in HNSCC. In order to decrease the antigenicity of Mab U36 and decrease the rate of HAMA, a chimeric antibody was constructed, with neither the chimeric nor the original murine Mab U36 exhibiting ADCC activity. However, the study found no evidence of anti-cancer activity of unlabeled Mab U36.

$^{186}$Re-labelled chimeric Mab U36 was used to determine the utility of Mab U36 as a therapeutic agent. In this Phase 1 escalating dose trial, 13 patients received a scouting dose of $^{99m}$Tc-labelled chimeric Mab U36 followed by $^{186}$Re-labelled chimeric Mab U36. No acute adverse events were reported. However, following treatment, dose limiting myelotoxcity (1.5 GBq/m$^2$) was noted in 2 of 3 patients, and thrombocytopenia was observed in one patient treated with the maximum tolerated dose (1.0 GBq/m$^2$). Although there were some effects on tumor size, these effects did not fulfill the criteria for objective responses to treatment. A further study of $^{186}$Re-labelled chimeric Mab U36 employed a strategy of using granulocyte colony-stimulating factor stimulated whole blood reinfusion to double the maximum-tolerated activity to 2.8 Gy. In this study of nine patients with various tumors of the head and neck, 3 required transfusions for drug related anemia. Other toxicity included grade 3 myelotoxicity and grade 2 mucositis. No objective tumor responses were reported, although stable disease was achieved for 3-5 months in 5 patients.

Due to the poor prognosis for HNSCC patients, the impact of the disease on quality of life, and the limited treatment options, there is considerable interest in, and a compelling need for, the development of new therapies directed to HNSCC. To date there have been no reports of a so-called "naked" (i.e. unconjugated) anti-CD44 antibody having efficacy in HNSCC. Thus, while CD44 appears to be a potential anti-cancer target for immunotherapy, anti-CD44 antibodies require a radio-immuno-conjugate to achieve anti-cancer effects. However, radio-labeled anti-CD44 studies have demonstrated unacceptable toxicity associated with the therapy in relation to the clinical effects achieved. Accordingly, there is a need for developing new HNSCC-specific therapies.

The technical problem underlying the present invention is the provision of methods and means for the prevention, treatment or diagnosis of HNSCC using anti-CD44 antibodies and, in some aspects particular, unlabeled anti-CD44 antibodies.

The technical problem is solved by provision of the embodiments characterized in the claims.

Herein described are novel methods for preventing, treating or diagnosing head and neck squamous cell carcinoma (HNSCC) comprising the use of anti-CD44 antibodies. The invention encompasses the use of anti-CD44 antibodies that bind to the amino terminal domain of the extracellular domain of CD44 and/or that immunospecifically bind to one or more, preferably multiple human variants CD44 as expressed in humans, for the treatment of HNSCC. Herein described is the use of the anti-CD44 antibody produced by the hybridoma deposited with the ATCC having accession number PTA-4621, and variants and/or derivatives thereof, e.g., chimeric and humanized versions of PTA-4621. Also described is the use of antibodies and antigen binding fragments thereof that compete for binding with PTA-4621. In particular the present invention relates to the use of a monoclonal anti-CD44 antibody, or antigen binding fragment thereof, for the manufacture of a medicament for the treatment of a head and neck squamous cell carcinoma (HNSCC) in a mammal, wherein said HNSCC is characterized by the expression of CD44, wherein said antibody comprises

(a) a $V_H$ CDR1 having the amino acid sequence of SEQ ID NO:3, a $V_H$ CDR2 having the amino acid sequence of SEQ ID NO:4, a $V_H$ CDR3 having the amino acid sequence of SEQ ID NO:5, a $V_L$ CDR1 having the amino acid sequence of SEQ ID NO:6, a $V_L$ CDR2 having the amino acid sequence of SEQ ID NO:7 and a $V_L$ CDR3 having the amino acid sequence of SEQ ID NO:8; or

(b) a $V_H$ domain having an amino acid sequence that is at least 85% identical to the $V_H$ domain having the amino acid sequence of SEQ ID NO:9 or SEQ NO:10 and a $V_L$ domain having an amino acid sequence that is at least 85% identical to the $V_L$ domain having the amino acid sequence of SEQ ID NO:11;

wherein said antibody competes for binding to CD44 with the antibody produced by the hybridoma deposited with the ATCC with Accession number PTA-4621. The invention relates also to a humanized anti-CD44 antibody, or antigen binding fragment thereof, for use in the treatment of a head and neck squamous cell carcinoma (HNSCC) in a human, wherein said HNSCC is characterized by the expression of CD44 and wherein said humanized antibody comprises a $V_H$ domain having the amino acid sequence of SEQ ID NO:9 and a $V_L$ domain having the amino acid sequence of SEQ ID NO:11, or comprises a $V_H$ domain having the amino acid sequence of SEQ ID NO:10 and a $V_L$ domain having the amino acid sequence of SEQ ID NO:11.

The antibodies and fragments used according to the methods are specific for CD44, in particular CD44 as expressed on the surface of a cell, and preferably, specific for the amino terminal extracellular domain of one or more variants of human CD44, e.g., as expressed on the surface of a HNSCC cancer cell. In certain aspects the antibodies for use according to the methods disclosed herein are humanized or chimeric versions of the antibody produced by the hybridoma deposited with the ATCC having accession number PTA-4621. The antibodies for use according to the methods disclosed herein, or antigen binding fragments thereof, comprise one or more of

a heavy chain variable domain ($V_H$) CDR1 having the amino acid sequence of RYWMS (SEQ ID NO:3),
a $V_H$ CDR2 having the amino acid sequence of EVNPDSTSINYTPSLKD (SEQ ID NO:4),
a $V_H$ CDR3 having the amino acid sequence of PNYYGSRYHYYAMDY (SEQ ID NO:5),
a light chain variable domain ($V_L$) CDR1 having the amino acid sequence of RASQDINNYLN (SEQ ID NO:6),
a $V_L$ CDR2 having the amino acid sequence of YTSRLHS (SEQ ID NO:7); and
a $V_L$ CDR3 having the amino acid sequence of QQGSTLPFT (SEQ ID NO:8).

The antibodies as used herein may also comprise two or more of, three or more of, four or more of, five or more of, and, in certain aspects, all of a $V_H$ CDR1 having the amino acid sequence of SEQ ID NO:3, a $V_H$ CDR2 having the amino acid sequence of SEQ ID NO:4, a $V_H$ CDR3 having the amino acid sequence of SEQ ID NO:5, a $V_L$ CDR1 having the amino acid sequence of SEQ ID NO:6, a $V_L$ CDR2 having the amino acid sequence of SEQ ID NO:7 and a $V_L$ CDR3 having the amino acid sequence of SEQ ID NO:8.

The present invention also provides for a monoclonal anti-CD44 antibody, or antigen binding fragment thereof, for use in the treatment of a head and neck squamous cell carcinoma (HNSCC) in a mammal, wherein said HNSCC is characterized by the expression of CD44, wherein said antibody comprises

(a) a $V_H$ CDR1 having the amino acid sequence of SEQ ID NO:3, a $V_H$ CDR2 having the amino acid sequence of SEQ ID NO:4, a $V_H$ CDR3 having the amino acid sequence of SEQ ID NO:5, a $V_L$ CDR1 having the amino acid sequence of SEQ ID NO:6, a $V_L$ CDR2 having the amino acid sequence of SEQ ID NO:7 and a $V_L$ CDR3 having the amino acid sequence of SEQ ID NO:8;
or
(b) a $V_H$ domain having an amino acid sequence that is at least 85% identical to the $V_H$ domain having the amino acid sequence of SEQ ID NO:9 or SEQ NO:10 and a $V_L$ domain having an amino acid sequence that is at least 85% identical to the $V_L$ domain having the amino acid sequence of SEQ ID NO:11;

wherein said antibody competes for binding to CD44 with the antibody produced by the hybridoma deposited with the ATCC with Accession number PTA-4621.

The antibodies and antigen binding fragments for use according to the methods of the invention may comprise

a $V_H$ domain having the amino acid sequence of

EVKLLESGGGLVQPGGSLKLSCATSGFDFSRYWMSWVRQAPGKGL
EWIGEVNPDSTSINYTPSLKDQFIISRDNAKNTLDLQMSKVSSEDTA
LYYCTRPNYYGSRYHYYAMDYWGQGTSVTVSS (SEQ ID NO:1)

and/or a $V_L$ domain having the amino acid sequence of

DIQMTQTTSSLSVSLGDRVTINCRASQDINNYLNWYQQKPDGTVKL
LIYYTSRLHSGVPSRFSGSGSGTDFSLTISNLEKEDVATYFCQQGSTL
PFTFGSGTKLEIK (SEQ ID NO:2).

In certain aspects the invention encompasses the use of an anti-CD44 antibody or antigen binding fragment comprising a $V_H$ domain having the amino acid sequence of SEQ ID NO:1 and a $V_L$ domain having the amino acid sequence of SEQ ID NO:2.

The invention further encompasses the use of an anti-CD44 antibody, or antigen binding fragments thereof, for treating, preventing or diagnosing an HNSCC, wherein the anti-CD44 antibody is humanized. In some aspects, the humanized anti-CD44 antibody or antigen binding fragment comprises

a $V_H$ domain having the amino acid sequence of

EVQLVESGGGLVQPGGSLRLSCAASGFDFSRYWMSWVRQAPGKGL
VWVGEVNPDSTSINYTPSLKDRFTISRDNAKNTLYLQMNSLRAEDT
AVYYCTRPNYYGSRYHYYAMDYWGQGTLVTVSS (SEQ ID NO:9)

or

EVQLVESGGGLVQPGGSLRLSCATSGFDFSRYWMSWVRQAPGKGL
VWIGEVNPDSTSINYTPSLKDQFTISRDNAKNTLYLQMNSLRAEDTA
VYYCTRPNYYGSRYHYYAMDYWGQGTLVTVSS (SEQ ID NO:10)

and/or a $V_L$ domain having the sequence of

DIQMTQSPSSLSASVGDRVTITCRASQDINNYLNWYQQKPGKAPKL
LIYYTSRLHSGVPSRFSGSGSGTDFTFTISSLQPEDIATYYCQQGSTLP
FTFGQGTKLEIK (SEQ ID NO:11).

In a particular aspect, the invention provides for a humanized anti-CD44 antibody or antigen binding fragment thereof for use in preventing, treating or diagnosis HNSCC in a subject, wherein said humanized anti-CD44 antibody comprises a $V_H$ domain having the amino acid sequence of SEQ ID NO:9 and a $V_L$ domain having the amino acid sequence of SEQ ID NO:11, or wherein said anti-CD44 antibody comprises a $V_H$ domain having the amino acid sequence of SEQ ID NO:10 and a $V_L$ domain having the amino acid sequence of SEQ ID NO:11. In particular aspects the subject is a human. The invention also provides for the use of a humanized anti-CD44 antibody or antigen binding fragment thereof for the prevention, treatment or diagnosis of HNSCC in a subject, wherein said humanized anti-CD44 antibody comprises a $V_H$ domain having the amino acid sequence of SEQ ID NO:9 and a $V_L$ domain having the amino acid sequence of SEQ ID NO:11, or wherein said anti-CD44 antibody comprises a $V_H$ domain having the amino acid sequence of SEQ ID NO:10 and a $V_L$ domain having the amino acid sequence of SEQ ID NO:11.

In certain aspects, the antibody or antibody fragment for use in accordance with the methods of the invention recognizes the epitope within the amino terminal domain of the extracellular region of CD44 having the amino acid sequence of AFDGPITITIV (SEQ ID NO:12).

The anti-CD44 antibodies for use according to the invention may be conjugated to active moieties such as therapeutic or reporter moieties. The anti-CD44 antibodies may also be conjugated to hematogeneous cells from the subject. In certain aspects the therapeutic moieties are moieties known in the art to be beneficial for the treatment, prevention or diagnosis of HNSCC, such as, but not limited to, a cytotoxin, an enzyme, a radioactive element, a cytokine, an antimetabolite or an interferon.

The invention also encompasses anti-CD44 antibodies or antigen binding fragments thereof for use as single agent therapies or in combination with one or more other chemotherapeutic or diagnostic agents and/or therapies. Accordingly, the invention encompasses anti-CD44 antibodies (e.g., humanized or chimeric antibodies), or antigen binding fragments thereof, for use in combination with a standard or experimental treatment regimen for HNSCC (e.g., chemotherapy, radioimmunotherapy, or radiotherapy). Examples of therapeutic agents that are particularly useful in combination with a CD44-specific antibody or an antigen-binding fragment thereof, according to the methods disclosed herein for the prevention, treatment, or diagnosis HNSCC, include, but are not limited to chemotherapeutics as known in the art, alkylating agents, antimetabolites, natural products, and hormones. The combination therapies as disclosed herein may enable lower dosages of an anti-CD44 antibody or an antigen-binding fragment thereof and/or less frequent administration of anti-CD44 antibody or an antigen-binding fragment thereof to a subject with a HNSCC, to achieve a therapeutic or prophylactic effect. According to the methods disclosed herein, the anti-CD44 antibodies or antigen-binding fragments thereof may be co-administered, concurrently administered, and/or sequentially administered with the one or more other chemotherapeutic or diagnostic agents, or may be administered in conjunction with radiation therapy or surgery. Administration of the antibodies, fragments and/ or additional agents or therapies, may be by any means known in the art to be suitable for delivery of the particular agent or therapy to the tumor site. Such administration may be parenteral (e.g., IV, intramuscular or subcutaneous administration), oral or, in certain aspects, direct delivery to the tumor site or to the site of suspected tumor occurrence or recurrence. The antibodies, fragments and/or compositions may also be administered as a sustained release formulation.

The invention further provides a pharmaceutical composition for the prevention, treatment or diagnosis of HNSCC in a subject comprising (i) a therapeutically effective amount of an anti-CD44 antibody as disclosed herein (e.g., a chimeric or humanized antibody), or an antigen binding fragment thereof; and (ii) a pharmaceutically acceptable carrier.

Also disclosed herein are methods of diagnosis of HNSCC in a subject comprising suspected of having such disease comprising: (i) contacting a biological sample from said subject with an effective amount of an anti-CD44 antibody as disclosed herein or antigen binding fragment thereof; and (ii) detecting binding of said antibody or a fragment thereof, wherein detection of said antibody or fragment above a background or standard level indicates that said subject has HNSCC. The detection of the anti-CD44 antibody or antigen binding fragment thereof may be aided by the use of a detectable marker as is known in the art. The disclosure also encompasses a diagnostic or prognostic kit for detection of HNSCC in a tissue sample from a subject known or suspected to comprise HNSCC cells. In certain aspects, the kit comprises a means for detecting CD44, in particular as expressed by HNSCC cells and/or a tissue sample from a subject known or suspected to contain HNSCC cells. The kit may comprise (1) the monoclonal antibody produced by the hybridoma deposited with the ATCC as accession number PTA-4621, (2) an antibody that competes for binding with the monoclonal antibody produced by the hybridoma deposited with the ATCC as accession number PTA-4621; (3) an antibody comprising one or more of a $V_H$ CDR1 having the amino acid sequence of SEQ ID NO:3, a $V_H$ CDR2 having the amino acid sequence of SEQ ID NO:4, a $V_H$ CDR3 having the amino acid sequence of SEQ ID NO:5, a $V_L$ CDR1 having the amino acid sequence of SEQ ID NO:6, a $V_L$ CDR2 having the amino acid sequence of SEQ ID NO:7 and a $V_L$ CDR3 having the amino acid sequence of SEQ ID NO:8; or (4) an antigen binding fragment of the antibody of (1), (2) or (3). The kit may also comprise one or more of: a substrate or container for holding a biological sample, reference standard(s) of biomarker(s) in solution or solid form (e.g., CD44 or peptide specifically bound by the antibody produced by antibody PTA-4621), one or more antibodies specific for the biomarkers (e.g., antibody PTA-4621, and antigen binding fragment thereof, or a variant or derivative thereof), and directions for carrying out detection assay(s) for the biomarkers with the contents of the kit.

It is further an objective of the disclosure to teach a method of treatment of HNSCC using antibody PTA-4621 or antigen-binding fragments thereof. It is another objective of the disclosure to teach the diagnosis of HNSCC using antibod PTA-4621 or antigen-binding fragments thereof. It is a further objective of the disclosure to teach the use of PTA-4621 or antigen-binding fragments thereof for the prognosis or staging of HNSCC. It is a further objective of the disclosure to teach the use of PTA-4621 or antigen-binding fragments thereof for the selection of a patient for the treatment of a HNSCC.

In certain aspects, the anti-CD44 antibodies and antigen binding fragments thereof for use according to the methods disclosed herein may comprise an amino acid sequence of a $V_H$ domain and/or $V_L$ domain that is at least 45%, at least 50%, at least 55%, at least 60%, at least 65%, at least 70%, at least 75%, at least 80%, at least 85%, at least 90%, at least 95%, or at least 99% identical to the amino acid sequence of the $V_H$ domain and/or $V_L$ domain of the mouse monoclonal antibody produced by the hybridoma deposited with the ATCC having accession number PTA-4621, provided that the anti-CD44 antibodies or fragments exhibit binding specificity to CD44 and/or compete for binding with PTA-4621. The invention also encompasses the use of anti-CD44 antibodies or antigen binding fragments thereof that comprise an amino acid sequence of a $V_H$ domain and/or $V_L$ domain that is at least 45%, at least 50%, at least 55%, at least 60%, at least 65%, at least 70%, at least 75%, at least 80%, at least 85%, at least 90%, at least 95%, or at least 99% identical to the $V_H$ domain having the amino acid sequence of SEQ ID NO:1 and/or the $V_L$ domain having the amino acid sequence of SEQ ID NO:2, provided that the anti-CD44 antibodies or fragments exhibit binding specificity to CD44 and/or compete for binding with PTA-4621. In certain aspects, the present invention encompasses antibodies or fragments thereof that specifically bind CD44 and that comprise an amino acid sequence of one or more CDRs that is at least 45%, at least 50%, at least 55%, at least 60%, at least 65%, at least 70%, at least 75%, at least 80%, at least 85%, at least 90%, at least 95%, or at least 99% identical to the amino acid sequence of one or more CDRs of the mouse monoclonal antibody produced by the hybridoma deposited with the ATCC having accession number PTA-4621, provided that said antibodies or fragments exhibit binding specificity to CD44 and/or compete for binding with PTA-4621. The invention also encompasses the use of anti-CD44 antibodies or antigen binding fragments thereof that comprise an amino acid sequence of one or more CDRs that are at least 45%, at least 50%, at least 55%, at least 60%, at least 65%, at least 70%, at least 75%, at least 80%, at least 85%, at least 90%, at least 95%, or at least 99% identical to one or more of a $V_H$ CDR1 having the amino acid sequence of SEQ ID NO:3, a $V_H$ CDR2 having the amino acid sequence of SEQ ID NO:4, a $V_H$ CDR3 having the amino acid sequence of SEQ ID NO:5, a $V_L$ CDR1 having the amino acid sequence of SEQ ID NO:6, a $V_L$ CDR2 having the amino acid sequence of SEQ ID NO:7 and a $V_L$ CDR3 having the amino acid sequence of SEQ ID NO:8, provided that provided that said antibodies or fragments exhibit binding specificity to CD44 and/or competes for binding with PTA-4621.

Disclosed herein is, accordingly, the use of humanized antibodies and antibody fragments specific for CD44 or that compete for binding with antibody PTA-4621, in which one or more regions of one or more CDRs of the heavy and/or light chain variable regions of a human antibody (the recipient antibody) have been substituted by analogous parts of one or more CDRs of a donor monoclonal antibody which specifically binds CD44, *e.g.*, of the murine monoclonal antibody produced by clone PTA-4621. Preferably, the humanized antibody specifically binds to the same epitope as the donor murine antibody. It will be appreciated by one skilled in the art that the invention encompasses CDR grafting of antibodies in general. In particular aspects, the present invention encompasses the use of humanized antibodies or antibody fragments that specifically bind CD44 and/or compete for binding with PTA-4621, which humanized antibodies or antibody fragments comprise an amino acid sequence of a $V_H$ domain and/or $V_L$ domain that is at least 45%, at least 50%, at least 55%, at least 60%, at least 65%, at least 70%, at least 75%, at least 80%, at least 85%, at least 90%, at least 95%, or at least 99% identical to the $V_H$ domain having the amino acid sequence of SEQ ID NO:9 or SEQ ID NO: 10 and/or the $V_L$ domain having the amino acid sequence of SEQ ID NO:11.

The anti-CD44 antibodies and antigen binding fragments thereof for use according to the methods disclosed herein may be encoded by a nucleotide sequence that hybridizes under stringent conditions to the nucleotide sequence encoding a $V_H$ domain, the complete heavy chain, a $V_L$ domain or the complete light chain of the murine monoclonal antibody PTA-4621. In other aspects, the invention encompasses the use of a an anti-CD44 antibody or antigen binding fragment thereof comprising a $V_H$ domain that is encoded by a nucleic acid sequence that hybridizes under stringent conditions to a nucleic acid sequence encoding the amino acid sequence of SEQ ID NO:1, SEQ ID NO:9 or SEQ ID NO:10. In other aspects, the invention encompasses the use of a an anti-CD44 antibody or antigen binding fragment thereof comprising a $V_L$ domain that is encoded by a nucleic acid sequence that hybridizes under stringent conditions to a nucleic acid sequence encoding the amino acid sequence of SEQ ID NO:2 or SEQ ID NO:11. The invention encompasses the use of a an anti-CD44 antibody or antigen binding fragment thereof comprising one or more CDRs encoded by a nucleotide sequence that hybridizes under stringent conditions to the nucleotide sequence encoding one or more CDRs of the murine monoclonal antibody PTA-4621, or the nucleic acid sequence encoding one or more of $V_H$ CDR1 having the amino acid sequence of SEQ ID NO:3, a $V_H$ CDR2 having the amino acid sequence of SEQ ID NO: 4, a $V_H$ CDR3 having the amino acid sequence of SEQ ID NO:5, a $V_L$ CDR1 having the amino acid sequence of SEQ ID NO:6, a $V_L$ CDR2 having the amino acid sequence of SEQ ID NO:7 and a $V_L$ CDR3 having the amino acid sequence of SEQ ID NO:8. Stringent hybridization conditions include, but are not limited to, (i) hybridization to filter-bound DNA in 6X sodium chloride/sodium citrate (SSC) at about 45°C, followed by one or more washes in 0.2X SSC/0.1% SDS at about 50-65°C; (ii) highly stringent conditions such as hybridization to filter-bound DNA in 6X SSC at about 45°C, followed by one or more washes in 0.1X SSC/0.2% SDS at about 60°C, or (iii) any other stringent hybridization conditions known to those skilled in the art.

Herein disclosed is also the use of a vector comprising the nucleic acids disclosed herein (*i.e.*, nucleic acids encoding $V_H$ domains, $V_L$ domains and/or one or more CDR of anti-CD44 antibody or antigen binding fragments). Said vector may be an expression vector and the nucleic acid sequences encoding the amino acid sequences as described herein are operably joined with nucleic acid regulatory sequences or sequences encoding amino acid regulatory sequences (*e.g.,* transcription, translation, translocation signals) necessary for the proper expression of the encoded amino acid sequences. Disclosed are also host cells containing the vectors or nucleotide sequences encoding the antibodies or antibody fragments disclosed herein.

The invention is also illustrated by the appended examples.


## DEFINITIONS


In general, the following words or phrases have the indicated definition when used in the summary, description, examples, and claims.

The term "antibody" is used in the broadest sense and specifically covers, for example, single monoclonal antibodies (including agonist, antagonist, and neutralizing antibodies, de-immunized, murine, chimeric, humanized or human antibodies), antibody compositions with polyepitopic specificity, single-chain antibodies, diabodies, triabodies, immuno-conjugates, synthetic antibodies, camelized antibodies, single-chain Fvs (scFv), single chain antibodies, Fab fragments, F(ab') fragments, disulfide-linked Fvs (sdFv), intrabodies, and anti-idiotypic (anti-Id) antibodies (including, e.g., anti-Id and anti-anti-Id antibodies to antibodies of the invention), and epitope-binding fragments of any of the above. In particular, antibodies include immunoglobulin molecules and immunologically active fragments of immunoglobulin molecules, i.e., molecules that contain an antigen binding site. The invention encompasses the use of immunoglobulin molecules of any type (e.g., IgG, IgE, IgM, IgD, IgA and IgY), class (e.g., $IgG_1$, $IgG_2$, $IgG_3$, $IgG_4$, $IgA_1$ and $IgA_2$) or subclass.

An "antibody fragment" or "antigen binding fragment" comprises a portion of an intact antibody, preferably comprising the antigen-or epitope-binding, or variable region thereof. Examples of antibody fragments include less than full length antibodies, e.g., Fab, Fab', $F(ab')_2$, as well as constructs of the antibody variable domains, e.g., Fv fragments; diabodies; linear antibodies; single-chain antibody molecules; single-chain antibodies, single domain antibody molecules, fusion proteins, recombinant proteins and multispecific antibodies formed from antibody fragment(s).

"Humanized" and/or "chimeric" forms of non-human (e.g. murine) antibodies/immunoglobulins refer to antibodies which comprising specific chimeric immunoglobulins, immunoglobulin chains or fragments thereof (such as Fv, Fab, Fab', $F(ab')_2$ or other antigen-binding subsequences of antibodies) which results in the decrease of a human anti-mouse antibody (HAMA), human anti-chimeric antibody (HACA) or a human anti-human antibody (HAHA) response, compared to the original antibody, and contain the requisite portions (e.g. CDR(s), antigen binding region(s), variable domain(s), etc.) derived from said non-human immunoglobulin, necessary to reproduce the desired effect, while simultaneously retaining binding characteristics which are comparable to said non-human immunoglobulin. For the most part, humanized antibodies are human immunoglobulins (recipient antibody) in which residues from the complementarity determining regions (CDRs) of the recipient antibody are replaced by residues from the CDRs of a non-human species (donor antibody) such as mouse, rat or rabbit having the desired specificity, affinity and capacity (e.g., specificity for CD44). In some instances, Fv framework region (FR) residues of the human immunoglobulin are replaced by corresponding non-human FR residues. Furthermore, the humanized antibody may comprise residues which are found neither in the recipient antibody nor in the imported CDR or FR sequences. These modifications are made to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR residues are those of a human immunoglobulin consensus sequence. The humanized antibody optimally also will comprise at least a portion of an immunoglobulin constant region (Fc), typically that of a human immunoglobulin.

As used herein, the term "hypervariable region" refers to the amino acid residues of an antibody which are responsible for antigen binding. The hypervariable region comprises amino acid residues from a "Complementarity Determining Region" or "CDR" (e.g.,. residues 24-34 (L1), 50-56 (L2) and 89-97 (L3) in the light chain variable domain and 31-35 (H1), 50-65 (H2) and 95-102 (H3) in the heavy chain variable domain according to Kabat et al., Sequences of Proteins of Immunological Interest, 5th Ed. Public Health Service, National Institutes of Health, Bethesda, Md. (1991)) and/or those residues from a "hypervariable loop" (i.e., residues 26-32 (L1), 50-52 (L2) and 91-96 (L3) in the light chain variable domain and 26-32 (H1), 53-55 (H2) and 96-101 (H3) in the heavy chain variable domain; Chothia and Lesk, 1987, J. Mol. Biol. 196:901-917). "Framework Region" or "FR" residues are those variable domain residues other than the hypervariable region residues as herein defined. As used herein, the terms "heavy chain variable domain," "$V_H$ domain" and/or "$V_H$" are used interchangeably and reference the hypervariable region (encompassing both the CDR and framework domains) of the heavy chain of an antibody; the terms "light chain variable domain," "$V_L$ domain" and/or "$V_L$" are used interchangeably and reference the hypervariable region (encompassing both the CDR and framework domains) of the heavy chain of an antibody.

Throughout the instant specification, hybridoma cell lines, as well as the monoclonal antibodies which are produced therefrom, are referenced by the ATCC accession number PTA-4621. The internal designation of this antibody is ARH460-16-2. Therefore, reference to PTA-4621 may reference the hybridoma cell clone deposited with the ATCC that produces an anti-CD44 antibody, or may reference the anti-CD44 produced by the hybridoma itself. Reference to PTA-4621 also refers to the antibody with internal designation ARH460-16-2. Accordingly, as used herein, chimeric versions of PTA-4621 may be referenced as "chPTA-4621" or "chARH460-16-2," and humanized versions of PTA-4621 may be referenced as "huPTA-4621" or "huARH460-16-2." One of skill in the art will immediately recognize from context whether the antibody or the hybridoma clone is referenced. The murine monoclonal anti-CD44 antibody PTA-4621 was discovered using the methodology for producing patient-specific anti-cancer antibodies taught in U.S. Patent 6,180,357. Using the process, mice were immunized with cells from both a patient's lung tumor biopsy and the NCI-H460 lung cancer cell line. The antibody is disclosed in US Patent 7,252,821 and the hybridoma cell line expressing the antibody was deposited with the American Type Culture Collection, 10801 University Blvd., Manassas, VA 20110-2209 on September 4, 2002, under accession number PTA-4621. This antibody has demonstrated significant anti-tumor effects in a number of pre-clinical xenograft models, including both preventative and established in vivo models of human breast cancer (as disclosed in US Patent 7,252,821), in liver cancer (as disclosed in US Patent 8,048,416) and in prostate cancer (as disclosed in US Patent 7,507,537). In addition, PTA-4621 treatment in combination with a chemotherapeutic drug (Cisplatin) demonstrated anti-tumor activity in an established *in vivo* prostate cancer model (*ibid*). Chimeric and humanized versions of the murine antibody PTA-4621/ARH460-16-2 are disclosed in US Patent 8,071,072.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma (murine or human) method first described by Kohler et al., Nature, 256:495 (1975), or may be made by recombinant DNA methods (see, e.g., U.S. Pat. No.4,816,567). The "monoclonal antibodies" may also be isolated from phage antibody libraries using the techniques described in Clackson et al., Nature, 352:624-628 (1991) and Marks et al., J. Mol. Biol., 222:581-597 (1991).

A "cancerous disease modifying antibody" (CDMAB) refers to a monoclonal antibody which modifies the cancerous disease process in a manner which is beneficial to the patient, for example by reducing tumor burden or prolonging survival of tumor bearing individuals, and antibody-ligands thereof.

An antibody or antibody fragment that "binds" or "specifically binds" an antigen of interest, *e.g.* CD44, is one capable of binding that antigen with sufficient affinity such that the antibody is useful as a therapeutic or diagnostic agent in targeting a cell expressing the antigen. Where the antibody is one which binds CD44, it will usually preferentially bind CD44 as opposed to other receptors, and does not include incidental binding such as non-specific Fc contact, or binding to post-translational modifications common to other antigens and may be one which does not significantly cross-react with other proteins. Methods for the detection of an antibody that binds an antigen of interest are well known in the art and can include but are not limited to assays such as FACS, cell ELISA and Western blot.

In preferred embodiments, the antibodies or antibody fragments for use in accordance with the methods of the invention specifically bind CD44, in particular, as expressed on HNSCC, and/or compete for binding with murine monoclonal antibody PTA-4621 to CD44 using standard methods as known in the art. CD44 mediates a direct link between the extracellular matrix and the cytoskeleton via their conserved extracellular HA binding regions and intracellular ankyrin binding regions. CD44 proteins are also released in soluble form (solCD44) via proteases and are detectable in normal circulation and saliva. The CD44 receptor comprises a family of isoforms expressed in many cell types. These isoforms arise from alternative splicing of a region of variable exons (exons 5-14) present in CD44 mRNA, resulting in differing "stems" connecting the amino terminal domain of the extracellular region to the transmembrane domain. Isoforms are found in normal cells as CD44 standard (CD44s), CD44 epithelial (CD44E) or CD44v8-10, and CD4v3-10 in keratinocytes. Other CD44 variant isoforms (CD44v) are differentially expressed in some tumors. In particular aspects, the antibodies and antigen-binding fragments thereof for use in accordance with the methods of the invention immunospecfically bind the amino terminal domain of the extracellular region of CD44, thereby exhibiting specificity to multiple, and preferably all, functional isoforms of CD44 expressed in humans. In particular aspects, the invention encompasses the use of antibodies and antigen binding fragments thereof that specifically bind CD44 as expressed by HNSCC. In certain aspects, the antibodies and antigen-binding fragments for use in according to the methods of the present invention bind to the epitope of the amino terminal domain of the extracellular region of CD44 having the amino acid sequence of AFDGPITITIV (SEQ ID NO:12). Preferably, the antibodies for use according to the methods disclosed herein compete for binding to CD44 with PTA-4621 as determined by standard methods known in the art.

As used herein, the expressions "cell", "cell line", and "cell culture" are used interchangeably, and all such designations include progeny. It is also understood that all progeny may not be precisely identical in DNA content, due to deliberate or inadvertent mutations. Mutant progeny that have the same function or biological activity as screened for in the originally transformed cell are included. It will be clear from the context where distinct designations are intended.

As used herein, the phrase "administration directly to the cancer or tumor site" and analogous phrases refer to direct or substantially direct introduction including, without limitation, single or multiple injections of the antibodies, fragments or compositions directly into the tumor or peritumorally, continuous or discontinuous perfusion into the tumor or peritumorally, introduction of a reservoir into the tumor or peritumorally, introduction of a slow-release apparatus into the tumor or peritumorally, introduction of a slow-release formulation into the tumor or peritumorally, direct application onto the tumor, direct injection into an artery that substantially directly feeds the area of the tumor, direct injection into a lymphatic vessel that substantially drains into the area of the tumor, direct or substantially direct introduction in a substantially enclosed cavity (*e.g.,* pleural cavity) or lumen (*e.g.,* intravesicular). "Peritumoral" is a term that describes a region, within about 10 cm, preferably within 5 cm, more preferably within 1 cm, of what is regarded as the tumor boundary, such as, but not limited to, a palpable tumor border. "Direct administration" in the context of prevention of occurrence or prevention of recurrence is defined as administration directly into a site at risk for development or recurrence of a cancer.

As used herein, the terms "nucleic acids," "nucleic acid sequence" and "nucleotide sequences" include DNA molecules (*e.g.,* cDNA or genomic DNA), RNA molecules (e.g., mRNA), combinations of DNA and RNA molecules or hybrid DNA/RNA molecules, and analogs of DNA or RNA molecules. Such analogs can be generated using, for example, nucleotide analogs, which include, but are not limited to, inosine or tritylated bases. Such analogs can also comprise DNA or RNA molecules comprising modified backbones that lend beneficial attributes to the molecules such as, for example, nuclease resistance or an increased ability to cross cellular membranes. The nucleic acids or nucleotide sequences can be single-stranded, double-stranded, may contain both single-stranded and double-stranded portions, and may contain triple-stranded portions, but preferably is double-stranded DNA.

The term "hybridization" or "hybridizes" as used herein may relate to hybridizations under stringent or non-stringent conditions. Said hybridization conditions may be established according to conventional protocols described, *e.g.*, in Sambrook, Russell; "Molecular Cloning, A Laboratory Manual", Cold Spring Harbor Laboratory, N.Y. (2001); Ausubel; "Current Protocols in Molecular Biology", Green Publishing Associates and Wiley Interscience, N.Y. (1989), or Higgins and Hames (Eds.); "Nucleic acid hybridization, a practical approach" IRL Press Oxford, Washington DC, (1985). The setting of conditions is well within the skill of the artisan and can be determined according to protocols described in the art. Thus, the detection of only specifically hybridizing sequences will usually require stringent hybridization and washing conditions such as 0.1 x SSC, 0.1% SDS at 65 °C. Non-stringent hybridization conditions for the detection of homologous or not exactly complementary sequences may be set at 6 x SSC, 1% SDS at 65°C. As is well known, the length of the probe and the composition of the nucleic acid to be determined constitute further parameters of the hybridization conditions. Note that variations in the above conditions may be accomplished according to methods routine in the art through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Hybridizing nucleic acid molecules also comprise fragments of the above described molecules. Such fragments may represent nucleic acid sequences which code for WAVE1 or a functional fragment thereof which have a length of at least 12 nucleotides, preferably at least 15, more preferably at least 18, more preferably of at least 21 nucleotides, more preferably at least 30 nucleotides, even more preferably at least 40 nucleotides and most preferably at least 60 nucleotides. Furthermore, nucleic acid molecules which hybridize with any of the aforementioned nucleic acid molecules also include complementary fragments, derivatives and allelic variants of these molecules. Additionally, a hybridization complex refers to a complex between two nucleic acid sequences by virtue of the formation of hydrogen bonds between complementary G and C bases and between complementary A and T bases; these hydrogen bonds may be further stabilized by base stacking interactions. The two complementary nucleic acid sequences hydrogen bond in an anti-parallel configuration. A hybridization complex may be formed in solution (*e.g.,* Cot or Rot analysis) or between one nucleic acid sequence present in solution and another nucleic acid sequence immobilized on a solid support (*e.g.,* membranes, filters, chips, pins or glass slides to which, e.g., cells have been fixed). The terms "complementary" or "complementarity" refer to the natural binding of polynucleotides under permissive salt and temperature conditions by base-pairing. For example, the sequence "A-G-T" binds to the complementary sequence "T-C-A". Complementarity between two single-stranded molecules may be "partial", in which only some of the nucleic acids bind, or it may be complete when total complementarity exists between single-stranded molecules. The degree of complementarity between nucleic acid strands has significant effects on the efficiency and strength of hybridization between nucleic acid strands. This is of particular importance in amplification reactions, which depend upon binding between nucleic acids strands.

The term "hybridizing sequences" preferably refers to sequences which display a sequence identity of at least 40%, preferably at least 50%, more preferably at least 60%, even more preferably at least 70%, particularly preferred at least 80%, more particularly preferred at least 90%, even more particularly preferred at least 95% and most preferably at least 97% identity with a nucleic acid sequence as described above (*i.e.* SEQ ID NOs: 1-11) encoding the $V_H$ domain or $V_L$ domain of anti-CD44 antibodies or antigen binding fragments according to the invention, and/or CDRs of such anti-CD44 antibodies or fragments.

In accordance with the present invention, the term "identical" or "percent identity" in the context of two or more nucleic acid or amino acid sequences, refers to two or more sequences or subsequences that are the same, or that have a specified percentage of amino acid residues or nucleotides that are the same (*e.g.,* 60% or 65% identity, preferably, 70-95% identity, more preferably at least 95% identity with the amino acid sequences of, *e.g.,* SEQ ID NOs: 1-11 or the nucleic acid sequences encoding the amino acid sequences of SEQ ID NO:1-11) when compared and aligned for maximum correspondence over a window of comparison, or over a designated region as measured using a sequence comparison algorithm as known in the art, or by manual alignment and visual inspection. Sequences having, for example, 60% to 95% or greater sequence identity are considered to be substantially identical. Such a definition also applies to the complement of a test sequence. Those having skill in the art will know how to determine percent identity between/among sequences using, for example, algorithms such as those based on CLUSTALW computer program (Thompson, Nucl. Acids Res. 2(1994) 4673-4680) or FASTDB (Brutlag, Comp. App. Biosci. 6(1990) 237-245), as known in the art.

Although the FASTDB algorithm typically does not consider internal non-matching deletions or additions in sequences, *i.e.,* gaps, in its calculation, this can be corrected manually to avoid an overestimation of the % identity. CLUSTALW, however, does take sequence gaps into account in its identity calculations. Also available to those having skill in this art are the BLAST and BLAST 2.0 algorithms (Altschul, Nucl. Acids Res. 25(1997) 3389-3402; Altschul, J. Mol. Evol. 36(1993) 290-300; Altschul, J. Mol. Biol. 215(1990) 403-410). The BLASTN program for nucleic acid sequences uses as defaults a word length (W) of 11, an expectation (E) of 10, M=5, N=4, and a comparison of both strands. For amino acid sequences, the BLASTP program uses as defaults a wordlength (W) of 3, and an expectation (E) of 10. The BLOSUM62 scoring matrix (Henikoff, PNAS 89(1989) 10915) uses alignments (B) of 50, expectation (E) of 10, M=5, N=4, and a comparison of both strands.

Also described herein are nucleic acid molecules whose sequence is being degenerate in comparison with the sequence of an above-described hybridizing molecule. When used in accordance with the present invention the term "being degenerate as a result of the genetic code" means that due to the redundancy of the genetic code different nucleotide sequences code for the same amino acid.

Analogous computer techniques using BLAST are used to search for identical or related molecules in nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score which is defined as:

$$\frac{\% \text{ sequence identity } \times \% \text{ maximum BLAST score}}{100}$$

and it takes into account both the degree of similarity between two sequences and the length of the sequence match. For example, with a product score of 40, the match will be exact within a 1-2% error; and at 70, the match will be exact. Similar molecules are usually identified by selecting those which show product scores between 15 and 40, although lower scores may identify related molecules. Another example for a program capable of generating sequence alignments is the CLUSTALW computer program (Thompson, Nucl. Acids Res. 2(1994) 4673-4680) or FASTDB (Brutlag, Comp. App. Biosci. 6(1990) 237-245), as known in the art.

"Treatment or treating" refers to both therapeutic treatment and prophylactic or preventative measures, wherein the object is to prevent ameliorate or slow down (lessen) the targeted pathologic condition or disorder (*e.g.*, HNSCC), or one or more symptom associated therewith. The terms are also used herein to denote delaying the onset of, inhibiting (*e.g.* reducing or arresting the growth of), alleviating the effects of, or prolonging the life of a patient suffering from, cancer, in particular, HNSCC. Those in need of treatment include those diagnosed with the disorder, those suspected of having the disorder, those predisposed to have the disorder as well as those in whom the disorder is to be prevented. Hence, the mammal to be treated herein may have been diagnosed as having the disorder or may be predisposed or susceptible to the disorder. In some embodiments, treatment refers to the eradication, removal, modification, or control of primary, regional, or metastatic cancer tissue that results from the administration of one or more therapeutic agents according to the methods of the invention. In other embodiments, such terms refer to the minimizing or delaying the spread of cancer resulting from the administration of one or more therapeutic agents to a subject with such a disease. In other embodiments, such terms refer to elimination of disease causing cells.

As used herein, the terms "prevent", "preventing" and "prevention" refer to the prevention of the occurrence and/or recurrence or onset of one or more symptoms of a cancer disease in a subject resulting from the administration of a prophylactic or therapeutic agent.

Individuals considered at risk for developing cancer may benefit particularly from the invention, primarily because prophylactic treatment can begin before there is any evidence of the disorder (for example, premalignancy ("precancer") or dysplasia lesions which may or may not be visible to the naked eye, but harbor histologic premalignant changes). Individuals "at risk" include, *e.g.,* individuals exposed to carcinogens, *e.g.*, by consumption, *e.g.,* by inhalation and/or ingestion, at levels that have been shown statistically to promote cancer in susceptible individuals. Also included are individuals at risk due to exposure to ultraviolet radiation, or their environment, occupation, and/or heredity, as well as those who show signs of a precancerous condition such as polyps. Similarly, individuals in very early stages of cancer or development of metastases (*i.e.*, only one or a few aberrant cells are present in the individual's body or at a particular site in an individual's tissue) may benefit from such prophylactic treatment.

The terms "effective amount" and "effective to treat," as used herein, refer to an amount or concentration of antibody utilized for a period of time (including acute or chronic administration and periodic or continuous administration) that is effective within the context of its administration for causing an intended effect or physiological outcome. Effective amounts of antibody for use in the present invention include, for example, amounts that inhibit the growth of cancer, *e.g.,* tumors and/or tumor cells, improve the outcome for a patient suffering from or at risk for cancer, and improve the outcome of other cancer treatments.

The terms "patient" and "subject" are used interchangeably and are used throughout the specification to describe an animal, human or non-human, to whom treatment or diagnosis according to the methods of the present invention is provided.

The terms "head and neck squamous cell carcinoma" or HNSCC include, but are not limited to, cancers of the mouth, lip, nasal cavity, paranasal sinuses, pharynx, larynx, nasopharynx, throat and trachea.

A "chemotherapeutic agent" is a chemical compound useful in the treatment of cancer.

"Mammal" for purposes of treatment refers to any animal classified as a mammal, including humans, mice, SCID or nude mice or strains of mice, domestic and farm animals, and zoo, sports, or pet animals, such as sheep, dogs, horses, cats, cows, etc. Preferably, the mammal herein is human.

As used herein, the term "in combination" refers to the use of more than one prophylactic and/or therapeutic agents, *e.g.,* a prophylactic or therapeutic agent in addition the anti-CD44 antibodies or antigen binding fragments thereof as described herein. The use of the term "in combination" does not restrict the order in which prophylactic and/or therapeutic agents are administered to a subject with a disorder, *e.g.,* HNSCC. A first prophylactic or therapeutic agent can be administered prior to (*e.g.,* 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g.,* 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second prophylactic or therapeutic agent to a subject which had, has, or is susceptible to a disorder. The prophylactic or therapeutic agents are administered to a subject in a sequence and within a time interval such that the agent of the invention can act together with the other agent to provide an increased benefit than if they were administered otherwise. Any additional prophylactic or therapeutic agent can be administered in any order with the other additional prophylactic or therapeutic agents. "In combination" may also refer to the use of anti-CD44 antibodies or antigen-binding fragments thereof as disclosed herein in conjunction with standard anti-cancer treatment modalities as known in the art, *e.g.,* radiotherapy or surgery.

As used herein, an "immuno-conjugate" means any molecule or CDMAB such as an antibody chemically or biologically linked to cytotoxins, radioactive agents, cytokines, interferons, target or reporter moieties, enzymes, toxins, anti-tumor drugs or therapeutic agents. The antibody or CDMAB may be linked to the cytotoxin, radioactive agent, cytokine, interferon, target or reporter moiety, enzyme, toxin, anti-tumor drug or therapeutic agent at any location along the molecule so long as it is able to bind its target. Examples of immuno-conjugates include antibody toxin chemical conjugates and antibody-toxin fusion proteins. Immunoconjugates may find particular use as diagnostic agents.

Radioactive agents suitable for use as anti-tumor agents and/or in connection with the diagnostic methods as disclosed herein are known to those skilled in the art. For example, $^{131}$I or $^{211}$At may be used. These isotopes are attached to the antibody using conventional techniques (*e.g.* Pedley et al., Br. J. Cancer 68, 69-73 (1993)). Alternatively, the anti-tumor agent which is attached to the antibody is an enzyme which activates a prodrug. A prodrug may be administered which will remain in its inactive form until it reaches the tumor site where it is converted to its cytotoxin form once the antibody complex is administered. In practice, the antibody-enzyme conjugate is administered to the patient and allowed to localize in the region of the tissue to be treated. The prodrug is then administered to the patient so that conversion to the cytotoxic drug occurs in the region of the tissue to be treated. Alternatively, the anti-tumor agent conjugated to the antibody is a cytokine such as interleukin-2 (IL-2), interleukin-4 (IL-4) or tumor necrosis factor alpha (TNF-α). The antibody targets the cytokine to the tumor so that the cytokine mediates damage to or destruction of the tumor without affecting other tissues. The cytokine is fused to the antibody at the DNA level using conventional recombinant DNA techniques. Interferons may also be used.

**BRIEF DESCRIPTION OF THE FIGURES**

The figures show:

**Figure 1** shows representative FACS histograms of ARH460-16-2 antibodies directed against HNSCC cell lines T.Tn, SCC-15 and Detroit-562 (Fig. 1A) and CAL 27 (Fig. 1B), showing that ARH460-16-2 binds to such cell lines. In Fig. 1A, the antibody C225 (anti-EGFR) served as control; in Fig. 1B, the anti-CD20 antibody RITUXAN™ served as control.

**Figure 2** demonstrates the effect of chARH460-16-2 on tumor growth in an established human T.Tn HNSCC model. The vertical dashed lines indicate the period during which the antibody was intraperitoneally administered. Data points represent the mean +/- SEM.

**Figure 3**      demonstrates the effect of a chimeric PTA-4621 on mouse body weight in an established human T.Tn HNSCC model. Data points represent the mean +/- SEM.

**Figure 4**      demonstrates the effect of humanized PTA-4621 on tumor growth in an established SCC-15 HNSCC model. The vertical dashed lines indicate the period during which the antibody was intraperitoneally administered. Data points represent the mean +/- SEM.

**Figure 5**      demonstrates the effect of humanized PTA-4621 on mouse body weight in an established SCC-15 HNSCC model. Data points represent the mean +/- SEM.

**Figure 6**      demonstrates the effect of humanized PTA-4621 on tumor growth in an established Detroit 562 HNSCC model. The vertical dashed lines indicate the period during which the antibody was intraperitoneally administered. Data points represent the mean +/- SEM.

**Figure 7**      demonstrates the effect of humanized PTA-4621 on mouse body weight in an established Detroit 562 HNSCC model. Data points represent the mean +/- SEM.

**Figure 8**      demonstrates the effect of humanized PTA-4621 on relative tumor volume in an established CAL 27 HNSCC model.

**Figure 9**      demonstrates the effect of humanized PTA-4621 on tumor growth in an established CAL 27 HNSCC xenograft model. Tumor volume is presented as the group mean $\pm$ minus SEM. Vertical dashed lines indicate the first and last day of dosing.

**Figure 10**      demonstrates the effect of humanized PTA-4621 on mouse body weight in an established CAL 27 HNSCC model. Body weight is presented as the group mean $\pm$ SEM.

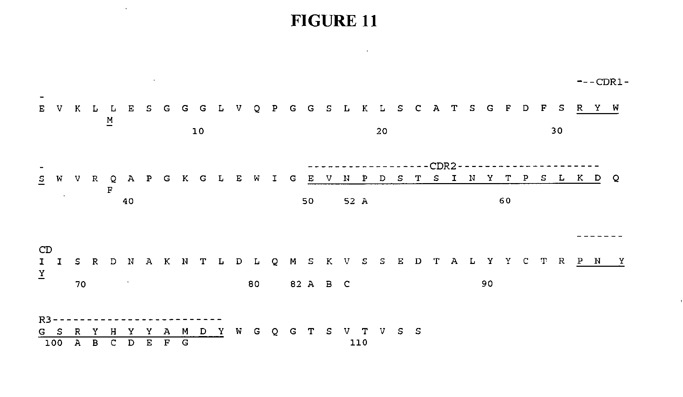

**Figure 11**      presents the amino acid sequence of the $V_H$ domain of the murine monoclonal antibody PTA-4621 (SEQ ID NO:1). The residues of the sequence have been numbered according to Kabat. The CDRs are underlined, and additionally identified with dashes above the sequence; the $V_H$ CDR1 (SEQ ID NO:3) corresponds to residues 31 to 35, the $V_H$ CDR2 (SEQ ID NO:4) corresponds to residues 50 to 65 and the $V_H$ CDR3 (SEQ ID NO:5) corresponds to residues 95 to 102.

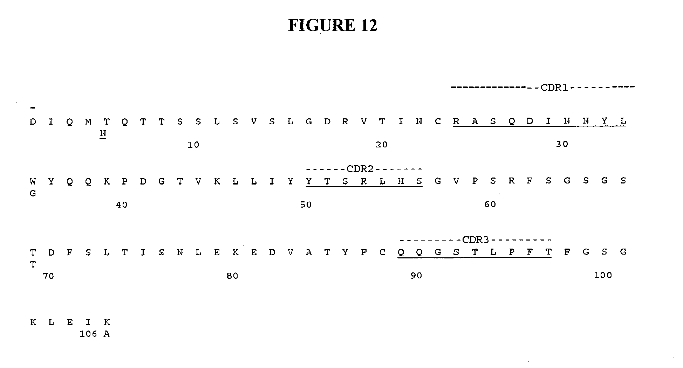

**Figure 12**      presents the amino acid sequence of the $V_L$ domain of the murine monoclonal antibody PTA-4621 (SEQ ID NO:2). The residues of the sequence have been numbered according to Kabat. The CDRs are underlined, and additionally identified with dashes above the sequence; the $V_L$ CDR1 (SEQ ID NO:6) corresponds to residues 24 to 34, the $V_L$ CDR2 (SEQ ID NO:7) corresponds to residues 50 to 67 and the $V_L$ CDR3 (SEQ ID NO:8) corresponds to residues 89 to 97.

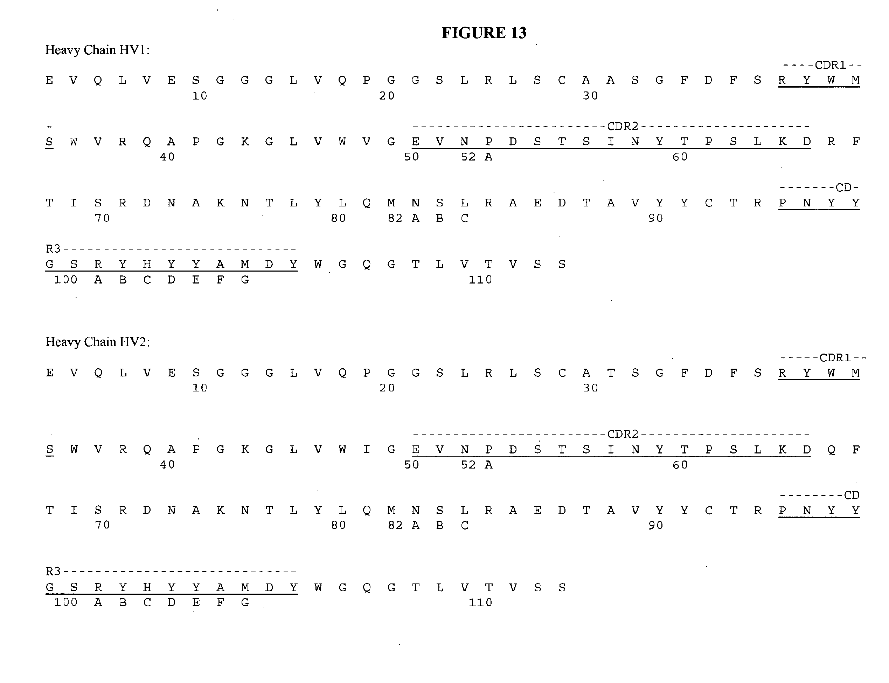

**Figure 13**      presents the amino acid sequences of two alternative humanized $V_H$ domains based on murine monoclonal antibody PTA-4621 (SEQ ID NO:9 and SEQ ID NO:10). The residues of the sequence have been numbered according to Kabat.

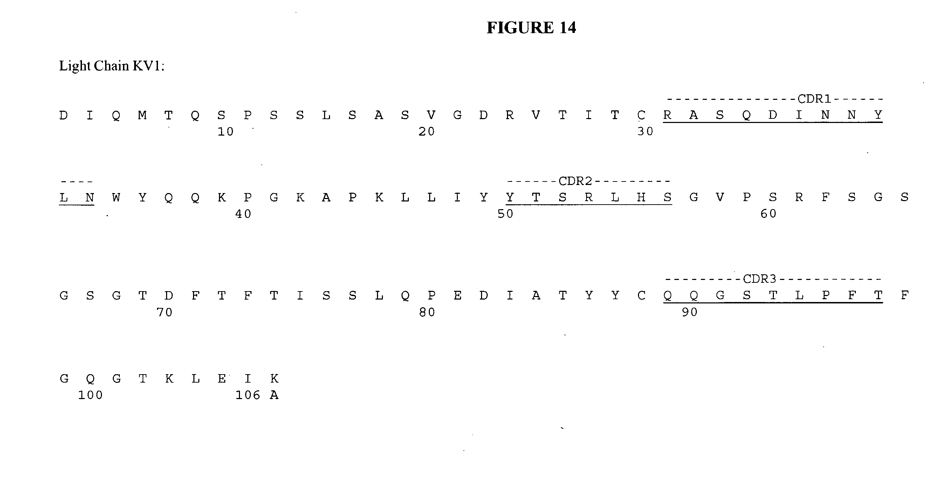

**Figure 14**      presents the amino acid sequence of a humanized $V_L$ domain based on murine monoclonal antibody PTA-4621 (SEQ ID NO:11). The residues of the sequence have been numbered according to Kabat.

## DETAILED DESCRIPTION

The present invention solves the identified technical problem in that it was surprisingly shown that the anti-CD44 antibody PTA-4621 is effective in the treatment of HNSCC as a naked (*i.e.*, unconjugated) antibody. Specifically, in contrast to previous anti-CD44 therapy in established models of HNSCC, unconjugated chimeric and humanized versions of PTA-4621 significantly inhibited and/or reduced tumor growth in HNSCC models comprising xenografts of T.Tn, SCC-15, Detroit 562 and Cal 27 cells.

Accordingly, the invention provides methods for preventing, treating and/or ameliorating the clinical condition of patients suffering from HNSCC comprising the use of anti-CD44 antibodies, in particular, anti-CD44 antibodies that compete for binding with murine monoclonal antibody PTA-4621 according to methods known in the art and/or anti-CD44 antibodies that immunospecifically bind to the amino terminal domain of the extracellular region of CD44 and recognize one or more isoforms of CD44 as expressed in humans. In certain aspects, the invention provides methods for (i) decreasing the HNSCC tumor size, growth rate, invasiveness, malignancy grade, and/or risk of recurrence, (ii) prolonging the disease-free interval following treatment, and/or (iii) improving breathing, swallowing, and/or speech function in a patient with HNSCC, comprising administering to the patient an effective amount of an anti-CD44 antibody as disclosed herein or an antigen binding fragment thereof. Clinical improvement may be subjectively or objectively determined, for example by evaluating the ability of a subject to breathe with less difficulty, the ability of the subject to swallow liquids versus solids, the degree of obstruction, the quality or volume of speech, and other indices known to the clinical arts.

Clinical outcomes of cancer treatments using the methods of the invention are readily discernible by one of skill in the relevant art, such as a physician. For example, standard medical tests to measure clinical markers of cancer may be strong indicators of the treatment's efficacy. Such tests may include, without limitation, physical examination, performance scales, disease markers, 12-lead ECG, tumor measurements, tissue biopsy, cytoscopy, cytology, longest diameter of tumor calculations, radiography, digital imaging of the tumor, vital signs, weight, recordation of adverse events, assessment of infectious episodes, assessment of concomitant medications, pain assessment, blood or serum chemistry, urinalysis, CT scan, and pharmacokinetic analysis. Furthermore, synergistic effects of a combination therapy comprising the anti-CD44 antibody, fragment or composition as disclosed herein according to the methods of the invention and another cancer therapeutic may be determined by comparative studies with patients undergoing monotherapy.

Particularly in the case of HNSCC, improvements in breathing, swallowing, speech, and certain quality of life measurements are readily ascertainable. Additionally, remission of HNSCC may be evaluated using criteria accepted by the skilled artisan, *e.g.,* Therasse et al., "New guidelines to evaluate the response to treatment in solid tumors. European Organization for Research and Treatment of Cancer, National Cancer Institute of the United States, National Cancer Institute of Canada," J Natl Cancer Inst. 92(2000) 205-16.

In certain embodiments, the cancer is amenable to treatment by direct administration of the anti-CD44 antibodies, fragments or compositions as disclosed herein. For example, a target tumor mass may be close to the surface of the skin. In another example, a diseased tissue may be encapsulated by a cyst, or is found in a substantially enclosed cavity including, without limitation, a lumen. The effective dose of the anti-CD44 antibody, fragment or composition as disclosed herein to be administered may vary according to the mode of administration. Direct administration (*e.g.,* intratumoral injection) requires much smaller total body doses of the disclosed anti-CD44 antibodies or fragments as compared to systemic, intravenous administration. It will be evident to the skilled artisan that local administration can result in lower body doses, and in those circumstances, and resulting low circulating plasma level of immunotoxin would be expected and desired. In such cases the anti-CD44 antibody or antigen binding fragment as disclosed herein may be administered intratumorally at a total dose per cycle equivalent to, or below the maximum tolerated dose established in a safety trial but the dosage is standardized in relation to the tumor volume. In certain embodiments, the dose will be administered in a volume not exceeding about 20-50% of the tumor volume.

The invention also provides methods for reducing the risk of post-surgical complications comprising administering an effective amount of an anti-CD44 antibody, fragment or composition as disclosed herein, before, during, or after surgery to treat HNSCC.

The invention also provides methods for preventing occurrence, preventing or delaying recurrence, or reducing the rate of recurrence of HNSCC comprising administering (*e.g.,* direct administration) to a patient in need thereof an effective amount of an anti-CD44 antibody, fragment or composition as disclosed herein. Direct administration of an anti-CD44 antibody, fragment or composition as disclosed herein to a patient in need of such treatment may result in reduced doses of another cancer therapeutic having clinically significant efficacy. Such efficacy of the reduced dose of the other cancer therapeutic may not be observed absent the methods of the invention. Accordingly, the present invention provides methods for treating a tumor or cancer comprising administering a reduced dose of one or more other cancer therapeutics.

The invention also provides methods for sensitizing a tumor or cancer to one or more other cancer therapeutics comprising administering an anti-CD44 antibody, fragment or composition as disclosed herein. In a nonlimiting embodiment, the other cancer therapeutic comprises a chemotherapeutic known for activity against HNSCC as known in the art. In another nonlimiting embodiment, the other cancer therapeutic comprises radiation. The other cancer therapeutic may be administered prior to, overlapping with, concurrently, and/or after administration of the anti-CD44 antibody, fragment or composition as disclosed herein. When administered concurrently, the anti-CD44 antibody, fragment or composition as disclosed herein and other cancer therapeutic may be administered in a single formulation or in separate formulations, and if separately, then optionally, by different modes of administration. Accordingly, the combination of one or more immunotoxins and one or more other cancer therapeutics may synergistically act to combat the tumor or cancer.

The anti-CD44 antibodies for use in the methods of the invention, or antigen binding fragments thereof, may comprise a $V_H$ domain having the amino acid sequence of SEQ ID NO:1, SEQ ID NO:9 or SEQ ID NO:10, a $V_L$ domain having the amino acid sequence of SEQ ID NO:2 or SEQ ID NO:11, and/or one or more of $V_H$ CDR1 having the amino acid sequence of SEQ ID NO:3, a $V_H$ CDR2 having the amino acid sequence of SEQ ID NO:4, a $V_H$ CDR3 having the amino acid sequence of SEQ ID NO:5, a $V_L$ CDR1 having the amino acid sequence of SEQ ID NO:6, a $V_L$ CDR2 having the amino acid sequence of SEQ ID NO:7 and a $V_L$ CDR3 having the amino acid sequence of SEQ ID NO:8. In a specific embodiment the invention encompasses the use of an anti-CD44 antibody or antigen binding fragment thereof comprising a $V_H$ domain having the amino acid sequence of SEQ ID NO:1 and a $V_L$ domain comprising the sequence of SEQ ID NO:2. In other embodiments the invention encompasses the use of an anti-CD44 antibody or antigen binding fragment thereof comprising a $V_H$ domain having the amino acid sequence of SEQ ID NO:9 and a $V_L$ domain comprising the sequence of SEQ ID NO:11, or comprising a $V_H$ domain having the amino acid sequence of SEQ ID NO:10 and a $V_L$ domain comprising the sequence of SEQ ID NO:11. In certain embodiments, the invention encompasses the use of an anti-CD44 antibody or antigen binding fragment thereof comprising each of a $V_H$ CDR1 having the amino acid sequence of SEQ ID NO:3, a $V_H$ CDR2 having the amino acid sequence of SEQ ID NO:4, a $V_H$ CDR3 having the amino acid sequence of SEQ ID NO:5, a $V_L$ CDR1 having the amino acid sequence of SEQ ID NO:6, a $V_L$ CDR2 having the amino acid sequence of SEQ ID NO:7 and a $V_L$ CDR3 having the amino acid sequence of SEQ ID NO:8.

The anti-CD44 antibodies or antigen binding fragment for use in the methods of the invention may be (i) characterized for specific binding to CD44, in particular, to the amino terminal domain of the extracellular region of CD44; (ii) characterized for specific binding to the epitope of the amino terminal domain of the extracellular region of CD44 having the amino acid sequence of AFDGPITITIV (SEQ ID NO:12); or (iii) characterized for competing for binding with the murine monoclonal antibody PTA-4621 using any immunological or biochemical based method known in the art for such characterizing including quantitating the interaction of the (specific) antibody to CD44 or an epitope thereof. Specific or competitive binding of an antibody of the invention to CD44 or an epitope thereof may be determined, for example, using immunological or biochemical based methods including, but not limited to, an ELISA assay, surface plasmon resonance assays, immunoprecipitation assay, affinity chromatography, and equilibrium dialysis. Immunoassays include, but are not limited to, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few. Such assays are routine and well known in the art.

Humanized antibodies of the invention may also be assayed using any surface plasmon resonance based assays known in the art for characterizing the kinetic parameters of the interaction of the antibody with an antigen, *e.g.,* CD44. Any SPR instrument commercially available can be used in the instant invention as is known in the art.

The invention further contemplates the use of CDMABs, *e.g.,* anti-CD44 antibodies or antigen binding fragments thereof, to which target or reporter moieties are linked. Target moieties are first members of binding pairs. Anti-tumor agents, for example, are conjugated to second members of such pairs and are thereby directed to the site where the antigen-binding protein is bound. A common example of such a binding pair is avidin and biotin. In a preferred embodiment, biotin is conjugated to the target antigen of the CDMAB of the present invention, and thereby provides a target for an anti-tumor agent or other moiety which is conjugated to avidin or streptavidin. Alternatively, biotin or another such moiety is linked to the target antigen of the CDMAB of the present invention and used as a reporter, for example in a diagnostic system where a detectable signal-producing agent is conjugated to avidin or streptavidin. Further, a CDMAB according to the methods of the invention may be conjugated to a therapeutic agent or drug moiety that modifies a given biological response. Therapeutic agents or drug moieties are not to be construed as limited to classical chemical therapeutic agents. For example, the drug moiety may be a protein or polypeptide possessing a desired biological activity. Such proteins may include, for example, a toxin (*e.g.,* abrin, ricin A, pseudomonas exotoxin, diphtheria toxin, ricin, gelonin), and enzyme, a protein (*e.g.,* tumor necrosis factor), an interferon (*e.g.,* α-interferon (IFN-α), β-interferon (IFN-β)), an apoptotic agent, a thrombotic agent, an anti-angiogenic agent (*e.g.,* angiostatin, endostatin), or a biological response modifier (*e.g.,* a lymphokine, macrophage colony stimulating factor or a growth factor), a protease, or a ribonuclease. The CDMAB for use according to the methods of the invention may also be conjugated to therapeutic moieties such as a radioactive materials or macrocyclic chelators useful for conjugating radiometal ions. A common example of a macrocyclic chelator is 1,4,7,10-tetraazacyclododecane-N,N',N'',N''-tetraacetic acid (DOTA) which can be attached to the antibody via a linker molecule. Such linker molecules are commonly known and routinely used in the art.

Labeled antibodies, in particular, the anti-CD44 antibodies or their antigen binding fragments as disclosed herein, may be used in the methods of the invention for diagnostic or prognostic purposes to detect, diagnose, or monitor cancer disease. Labels as described herein are detectable signals coupled to the anti-CD44 antibody, or antigen binding fragment for use, *e.g.,* in *in vivo* and *in vitro* diagnostic methods of the invention. The signal producing agent produces a measurable signal which is detectable by external means, usually the measurement of electromagnetic radiation. For the most part, the signal producing agent is an enzyme, or chromophore or radionuclide, or emits light by fluorescence, phosphorescence or chemiluminescence. Chromophores include dyes which absorb light in the ultraviolet or visible region, and can be substrates or degradation products of enzyme catalyzed reactions.

Moreover, included within the scope of the present invention is use of CDMABs *in vivo* and *in vitro* for investigative or diagnostic methods, which are well known in the art. In order to carry out the diagnostic methods as contemplated herein, the instant invention may further include kits, which contain CDMABs useful in the present invention. Such kits will be useful for identification of individuals at risk for certain type of cancers by detecting over-expression of the CDMAB's target antigen on cells of such individual.

Antibodies useful in the practice of the present invention can be used as a composition for preventing, treating or diagnosing cancer. These compositions have low-toxicity and can be administered as they are in the form of liquid preparations, or as pharmaceutical compositions of suitable preparations to human or mammals orally or parenterally (*e.g.,* intravascularly, intraperitoneally, subcutaneously, etc.). Antibodies useful in the practice of the present invention may be administered by themselves, or may be administered as appropriate compositions. A composition used for such administration may contain a pharmacologically acceptable carrier with the antibody or its salt, a diluent or excipient. Such a composition is provided in the form of pharmaceutical preparations suitable for oral or parenteral administration.

Examples of the composition for parenteral administration are injectable preparations, suppositories, etc. The injectable preparations may include dosage forms such as intravenous, subcutaneous, intracutaneous and intramuscular injections, drip infusions, intraarticular injections, etc. These injectable preparations may be prepared by methods publicly known. For example, the injectable preparations may be prepared by dissolving, suspending or emulsifying the antibody of the present invention or its salt in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (*e.g.,* ethanol), a polyalcohol (*e.g.,* propylene glycol, polyethylene glycol), a nonionic surfactant (*e.g.,* polysorbate 80, HCO-50 (polyoxyethylene (50 mols) adduct of hydrogenated castor oil)), etc. As the oily medium, there are employed, *e.g.,* sesame oil, soybean oil, etc., which may be used in combination with a solubilizing agent such as benzyl benzoate, benzyl alcohol, etc. The injection thus prepared is usually filled in an appropriate ampoule. The suppository used for rectal administration may be prepared by blending the antibody of the present invention or its salt with conventional bases for suppositories. The composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and may contain a vehicle, a diluent or excipient conventionally used in the field of pharmaceutical preparations. Examples of the vehicle or excipient for tablets are lactose, starch, sucrose, magnesium stearate, etc.

Advantageously, the compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc.

The dose of the aforesaid prophylactic/therapeutic agent comprising the antibody according to the methods of the present invention may vary depending upon subject to be administered, target disease, conditions, route of administration, etc. For example, when used for the purpose of treating/preventing, *e.g.,* HNSCC in an adult, it is advantageous to administer the antibody of the present invention intravenously in a dose of about 0.0001 mg/kg to 200 mg/kg or 0.0001 mg/kg to 100 mg/kg of the patient's body weight. In other aspects, the dosage administered to a patient is between 0.0001 mg/kg and 20 mg/kg, 0.0001 mg/kg and 10 mg/kg, 0.0001 mg/kg and 5 mg/kg, 0.0001 and 2 mg/kg, 0.0001 and 1 mg/kg, 0.0001 mg/kg and 0.75 mg/kg, 0.0001 mg/kg and 0.5 mg/kg, 0.0001 mg/kg to 0.25 mg/kg, 0.0001 to 0.15 mg/kg, 0.0001 to 0.10 mg/kg, 0.001 to 0.5 mg/kg, 0.01 to 0.25 mg/kg or 0.01 to 0.10 mg/kg of the patient's body weight. Generally, human antibodies have a longer half-life within the human body than antibodies from other species due to the immune response to the foreign polypeptides. Thus, lower dosages of human antibodies and less frequent administration is often possible. Further, the dosage and frequency of administration of antibodies of the invention or fragments thereof may be reduced by enhancing uptake and tissue penetration of the antibodies by modifications such as, for example, lipidation. In other aspects, the antibodies of the invention are used in combination with other therapeutic compositions and the dosage administered to a subject are lower than when said antibodies are used as a single agent therapy.

The dosage amounts and frequencies of administration provided herein are encompassed by the terms therapeutically effective and prophylactically effective. The dosage and frequency further will typically vary according to factors specific for each patient depending on the specific therapeutic or prophylactic agents administered, the severity, the route of administration, as well as age, body weight, response, and the past medical history of the patient. Suitable regimens can be selected by one skilled in the art by considering such factors and by following, for example, dosages reported in the literature and recommended in the Physician's Desk Reference (56.sup.th ed., 2002). The daily dosage of the antibody, fragment or composition of the invention can be administered as a single bolus dose or divided into multiple doses to be delivered over a 24 hour period. Alternatively, the total daily dosage may be administered over an extended period of time via, *e.g.,* an infusion, such that the total dosage is administered over 12 hours, 6 hours, 4 hours, 2 hours, 1.5 hours, 1.0 hours. 45 minutes, 30 minutes, 25 minutes, 20 minutes, 15 minutes, 10 minutes, 9 minutes, 8 minutes, 7 minutes, 6 minutes, 5 minutes, 4 minutes, 3 minutes, 2 minutes or 1 minute. When the condition is especially severe, the dose may be increased according to the condition.

The antibody for use according to the methods of the present invention may be administered as it stands or in the form of an appropriate composition. The composition used for the administration may contain a pharmacologically acceptable carrier with the aforesaid antibody or its salts, a diluent or excipient. Such a composition is provided in the form of pharmaceutical preparations suitable for oral or parenteral administration (*e.g.,* intravascular injection, subcutaneous injection, etc.). Each composition described above may further contain other active ingredients. Furthermore, the antibody of the present invention may be used in combination with other drugs, for example, alkylating agents (*e.g.,* cyclophosphamide, ifosfamide, etc.), metabolic antagonists (e.g., methotrexate, 5-fluorouracil, etc.), anti-tumor antibiotics (e.g., mitomycin, adriamycin, etc.), plant-derived anti-tumor agents (e.g., vincristine, vindesine, Taxol, etc.), cisplatin, carboplatin, etoposide, irinotecan, etc. The antibody of the present invention and the drugs described above may be administered simultaneously or at staggered times to the patient. Methods of administering a humanized antibody of the invention include, but are not limited to, parenteral administration (e.g., intradermal, intramuscular, intraperitoneal, intravenous and subcutaneous), epidural, and mucosal (e.g., intranasal and oral routes). In a specific embodiment, the antibodies of the invention are administered intramuscularly, intravenously, or subcutaneously. The compositions may be administered by any convenient route, for example, by infusion or bolus injection, by absorption through epithelial or mucocutaneous linings (*e.g.*, oral mucosa, rectal and intestinal mucosa, etc.) and may be administered together with other biologically active agents. Administration can be systemic or local. In addition, pulmonary administration can also be employed, e.g., by use of an inhaler or nebulizer, and formulation with an aerosolizing agent.

The chemotherapeutic agent/other antibody regimens utilized include any regimen believed to be optimally suitable for the treatment of the patient's condition. Different malignancies can require use of specific anti-tumor antibodies and specific chemotherapeutic agents, which will be determined on a patient to patient basis. In a preferred embodiment of the invention, chemotherapy is administered concurrently with or, more preferably, subsequent to antibody therapy. It should be emphasized, however, that the present invention is not limited to any particular method or route of administration.

All patents and publications mentioned in this specification are indicative of the levels of those skilled in the art to which the invention pertains.

One skilled in the art will readily appreciate that the present invention is well adapted to carry out the objects and obtain the ends and advantages mentioned, as well as those inherent therein. Any oligonucleotides, peptides, polypeptides, biologically related compounds, methods, procedures and techniques described herein are presently representative of the preferred embodiments, are intended to be exemplary and are not intended as limitations on the scope.

Also encompassed by the disclosure the following items:

Item 1: A method for the treatment of a head and neck squamous cell carcinoma (HNSCC) in a mammal, comprising administering to said mammal an anti-CD44 monoclonal antibody or an antigen-binding fragment thereof in an amount effective to result in a reduction of said mammal's tumor burden.

Item 2: The method of item 1 wherein the anti-CD44 monoclonal antibody is ARH460-16-2.

Item 3: The method of item 2 wherein said monoclonal antibody is conjugated to a cytotoxic moiety.

Item 4: The method of item 1 wherein said monoclonal antibody is a humanized version of the monoclonal antibody produced by the hybridoma deposited with the ATCC as accession number PTA-4621 or an antigen binding fragment produced from said humanized antibody.

Item 5: The method of item 1 wherein said monoclonal antibody is a chimeric version of the monoclonal antibody produced by the hybridoma deposited with the ATCC as accession number PTA-4621 or an antigen binding fragment produced from said chimeric antibody.

Item 6: The use of an anti-CD44 monoclonal antibody or antigen-binding fragment thereof for the treatment of HNSCC in a mammal, comprising administering to said mammal said anti-CD44 monoclonal antibody or antigen-binding fragment thereof in an amount effective to result in a reduction of said mammal's tumor burden.

Item 7: The use of item 6 wherein the anti-CD44 monoclonal antibody is ARH460-16-2.

Item 8: The use of item 7 wherein said monoclonal antibody is conjugated to a cytotoxic moiety.

Item 9: The use of item 6 wherein said monoclonal antibody is a humanized version of the monoclonal antibody produced by the hybridoma deposited with the ATCC as accession number PTA-4621.

Item 10: The use of item 6 wherein said monoclonal antibody is a chimeric version of the monoclonal antibody produced by the hybridoma deposited with the ATCC as accession number PTA-4621.

Item 11: A method for the treatment of HNSCC in a mammal comprising administering to said mammal an anti-CD44 monoclonal antibody or an antigen-binding fragment thereof; in conjunction with at least one chemotherapeutic agent in an amount effective to result in a reduction of said mammal's tumor burden.

Item 12. The method of item 13 wherein the anti-CD44 monoclonal antibody is ARH460-16-2.

(continued)

Item 13: The method of item 14 wherein said monoclonal antibody or CDMAB is conjugated to said chemotherapeutic agent.

Item 14: The method of item 15 wherein said chemotherapeutic agent is a cytotoxic moiety.

Item 15. The method of item 11 wherein said monoclonal antibody is a humanized version of the monoclonal antibody produced by the hybridoma deposited with the ATCC as accession number PTA-4621.

Item 16: The method of item 11 wherein said monoclonal antibody is a chimeric version of the monoclonal antibody produced by the hybridoma deposited with the ATCC as accession number PTA-4621.

Item 17: A composition effective for the treatment of HNSCC comprising in combination: an anti-CD44 monoclonal antibody produced by a hybridoma deposited with the ATCC as accession number PTA-4621 or an antigen-binding fragment thereof; and a requisite amount of a pharmacologically acceptable carrier; wherein said composition is effective for treating said HNSCC.

Item 18: The composition of item 17, further including a conjugate of said monoclonal antibody or an antigen binding fragment thereof with a member selected from the group consisting of cytotoxic moieties, enzymes, radioactive compounds, cytokines, interferons, target or reporter moieties and hematogenous cells.

Item 19: An assay kit for detecting the presence of HNSCC, wherein said HNSCC expresses at least one epitope of an antigen which specifically binds to the monoclonal antibody produced by the hybridoma deposited with the ATCC as accession number PTA-4621 or an antigen-binding fragment thereof, the kit comprising the monoclonal antibody produced by the hybridoma deposited with the ATCC as accession number PTA-4621 or an antigen-binding fragment thereof, is bound to a polypeptide whose presence, at a particular cut-off level, is diagnostic of said presence of said HNSCC.

In order that the invention herein described may be more fully understood, the following non-limiting examples are set forth.

**EXAMPLES**

**EXAMPLE 1**

***In vitro* Binding to HNSCC Cell Lines.**

Chimeric and humanized versions of PTA-4621 were developed as disclosed herein. Chimeric PTA-4621 ("chPTA-4621") comprises the $V_H$ and $V_L$ domains having the amino acid sequence of SEQ ID NO:1 and SEQ ID NO: 2, respectively. Two humanized version of PTA-4621 (huPTA-4621) were developed, having minor differences in the amino acid sequence of their heavy chain variable domains. huPTA-4621 comprises a $V_H$ domain having the amino acid sequence of SEQ ID NO:9 or SEQ ID NO:10 and a $V_L$ domain having the amino acid sequence of SEQ ID NO:11. The antigen binding affinity and avidity of the two versions of huPTA-4621 were indistinguishable using standard assays as known in the art.

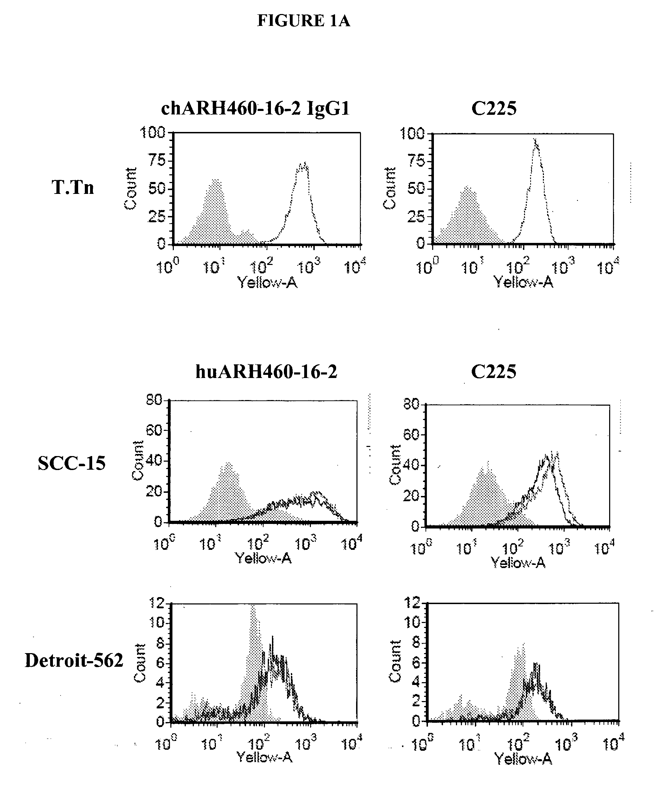

The binding of chPTA-4621 and huPTA-4621 to HNSCC cell lines T.Tn, SCC-15, Detroit-562 and CALL 27 was assessed by flow cytometry (FACS). Cells were prepared for FACS by initially washing the cell monolayer with DPBS (without $Ca^{++}$ and $Mg^{++}$). Either cell dissociation buffer (INVITROGEN, Burlington, ON; for T.Tn, SCC-15 and Detroit-562) or the cell detachment solution ACCUTASE™ (SIGMA, St. Lois, MO, USA, for CAL 27) was then used to dislodge the cells from their cell culture plates at 37 °C. After centrifugation and collection, the cells were resuspended in DPBS containing $MgCl_2$, $CaCl_2$ and 2 percent (T.Tn, SCC-15 and Detroit-562) or 3 percent (CAL 27) fetal bovine serum at 4°C (staining media) and counted, aliquoted to appropriate cell density, spun down to pellet the cells and resuspended in staining media at 4°C in the presence of test antibodies (chimeric or humanized PTA-4621) or control antibodies (isotype control, anti-EGFR (C225; T.Tn, SCC-15 and Detroit-562) or anti-CD20 (RITUXAN™; CAL27). Isotype control and test antibodies were assessed at 20 μg/mL on ice for 30 minutes. For detection of bound antibody, an Alexa Fluor 546 conjugated secondary antibody was used for T.Tn, SCC-15 and Detroit-562 cell ones and an APC conjugated secondary antibody was used in the CLA 27 tests. Prior to the addition of the secondary antibody, the cells were washed once with staining media. The secondary antibody in staining media was then added according to manufacturer's directions. The cells were then washed for the final time and resuspended in fixing media (staining media containing 1.5% paraformaldehyde). Flow cytometric acquisition of the T.Tn, SCC-15 and Detroit-562 cells was assessed by running samples on a FACScan using the CellQuest software (BD Biosciences, Oakville, ON), while acquisition of the CAL 27 cells was assessed by running samples on a LSR-II using the Diva 6 system Software (BD Biosciences, Rockville, MD USA). The forward (FSC) and side scatter (SSC) of the cells were set by adjusting the voltage and amplitude gains on the FSC and SSC detectors. The detectors for the fluorescence (FITC or APC) channel was adjusted by running cells unstained (CAL 27) or stained only with Alexa Fluor 546-conjugated secondary antibody (T.Tn, SCC-15 and Detroit-562) such that cells had a uniform peak with a median fluorescent intensity of approximately 1-5 units. For each sample, approximately 10,000 stained fixed cells were acquired for analysis and the results are presented in Figure 1A-B.

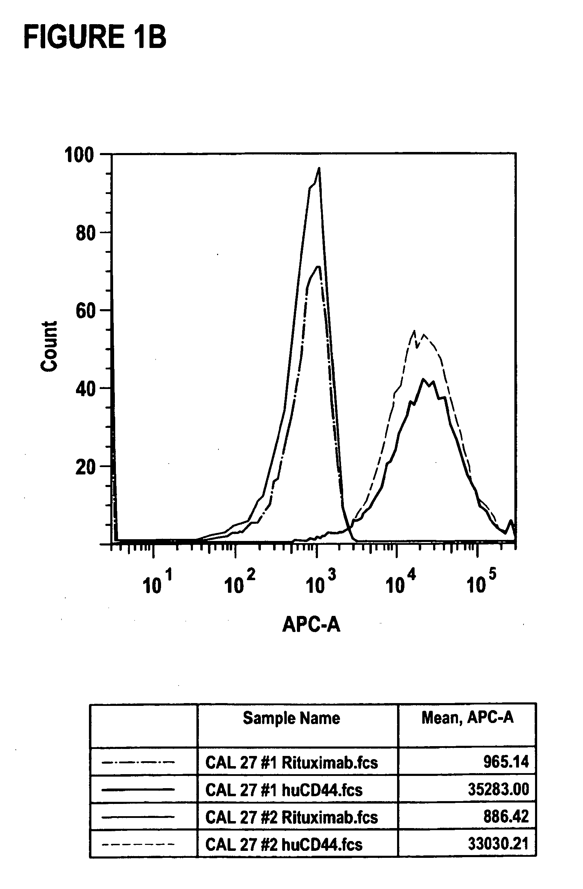

In summary, the data obtained shows that the chimeric and humanized versions of PTA-4621 bind to T.Tn, SCC-15 and Detroit-562 (Fig. 1A) and CAL 27 (Fig. 1B) cell lines.

## EXAMPLE 2

### *In vivo* Experiment with human T.Tn Cells

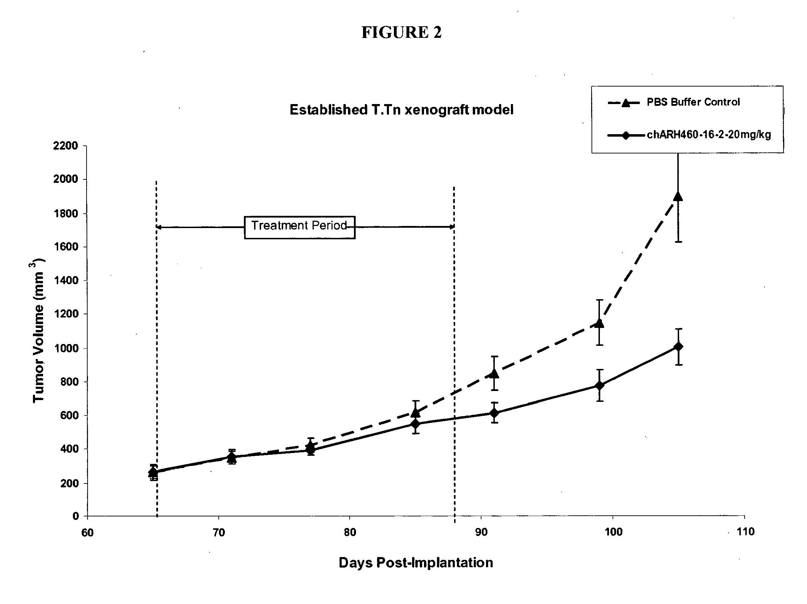

To demonstrate efficacy against a human HNSCC cancer cell line *in vivo,* chimeric PTA-4621 (chPTA-4621) was tested in an established T.Tn HNSCC xenograft model. With reference to Figures 2 and 3, 6 to 8 week old female SCID mice were implanted with 10 million T.Tn cells in 100 microliters PBS solution injected subcutaneously in the right flank. The mice were randomly divided into 2 treatment groups of 10. When the average tumor volume of the mice reached approximately 264-268 mm$^3$, 20 mg/kg of chPTA-4621 test antibody or buffer control was administered intraperitoneally to each cohort in a volume of 300 microliters after dilution from the stock concentration with a diluent that contained 2.7 mM KCl, 1 mM $KH_2PO_4$, 137 mM NaCl and 20 mM $Na_2HPO_4$. The antibody and control samples were then administered 3 times per week for the duration of the study. Tumor growth was measured about every 7 days with calipers. The study was completed after 10 doses of antibody. Body weights of the animals were recorded once per week for the duration of the study. At the end of the study all animals were euthanized according to CCAC guidelines.

chPTA-4621 reduced tumor growth in the T.Tn *in vivo* established model of human HNSCC. Treatment with antibody chPTA-4621 reduced the growth of T.Tn tumors by 54.9 percent (p=0.0068, t-test) compared to the buffer treated group, as determined on day 105, the last day of the study period (Figure 2).

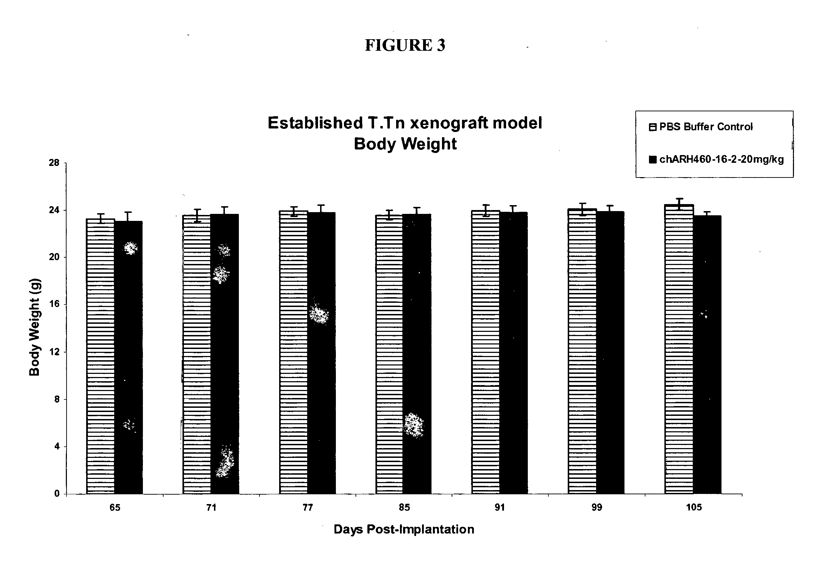

There were no clinical signs of toxicity throughout the study. Body weight measured at weekly intervals was a surrogate for well-being and failure to thrive (Figure 3). There was no significant difference in mean body weight between the groups at the end of the treatment period. There was also no significant difference in mean body weight within each group from the start to the end of the study.

In summary, chPTA-4621 was well-tolerated and significantly decreased the tumor burden in this human HNSCC xenograft model.

## EXAMPLE 3

### *In vivo* Experiment with human SCC-15 Cells

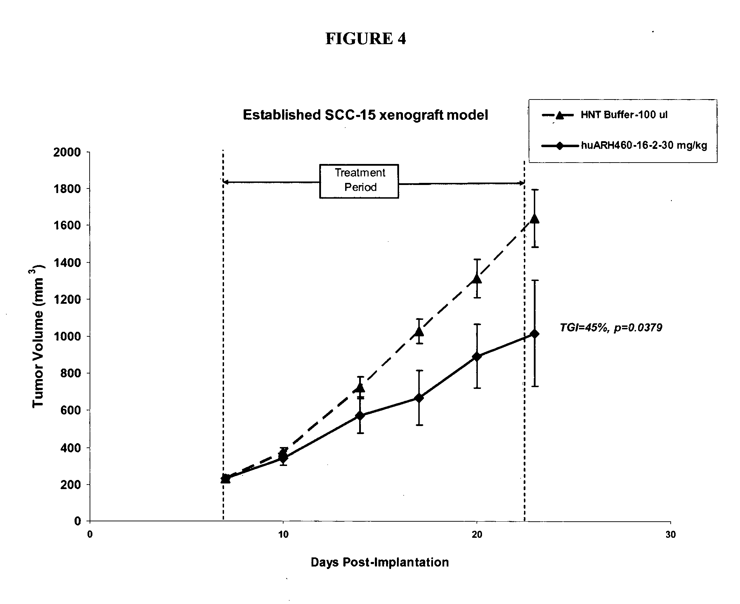

To demonstrate efficacy against a human HNSCC cancer cell line *in vivo,* humanized PTA-4621 (huPTA-4621) was tested in an SCC-15 HNSCC xenograft model. With reference to Figures 4 and 5, 6 to 8 week old female SCID mice were implanted with 3 million SCC-15 cells in 100 microliters PBS solution injected subcutaneously in the right flank. The mice were randomly divided into 2 treatment groups of 10. When the average tumor volume of the mice reached approximately 233-235mm$^3$, 30 mg/kg of huPTA-4621 test antibody or HNT buffer control was administered intraperitoneally to each cohort in a volume of 100 microliters after dilution from the stock concentration with a diluent that contained 20mM Histidine HCl, 150mM NaCl and 0.01% polysorbate 80, Ph 6.0. The antibody and control samples were then administered once per week for the duration of the study. Tumor growth was measured about every 7 day with calipers. The study was completed after 4 doses of antibody. Body weights of the animals were recorded once per week for the duration of the study. At the end of the study all animals were euthanized according to CCAC guidelines.

huPTA-4621 reduced tumor growth in the SCC-15 *in vivo* established model of human HNSCC. Treatment with antibody huPTA-4621 reduced the growth of SCC-15 tumors by 44.5 percent (p=0.0379, t-test) compared to the buffer treated group, as determined on day 23, the last day of the study period (Figure 4).

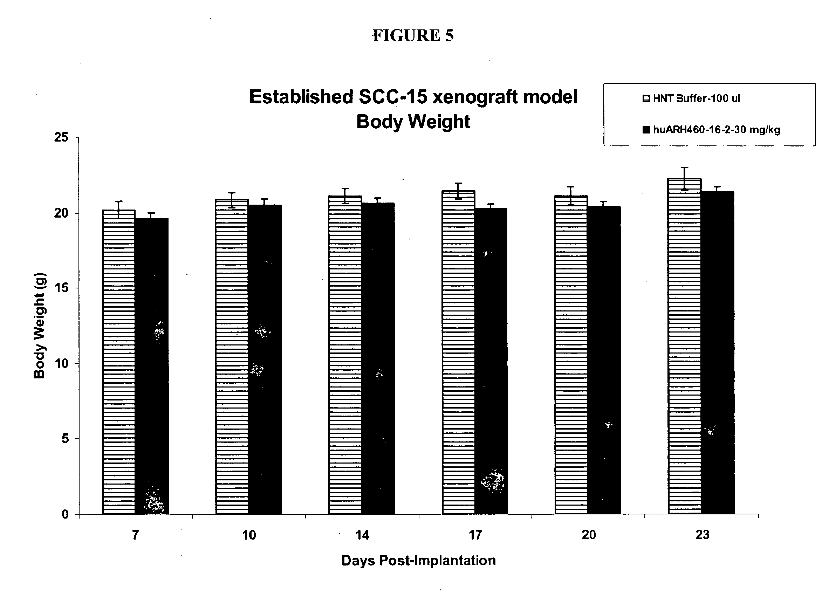

There were no clinical signs of toxicity throughout the study. Body weight measured at weekly intervals was a surrogate for well-being and failure to thrive (Figure 5). There was no significant difference in mean body weight between the groups at the end of the treatment period. There was also no significant difference in mean body weight within each group from the start to the end of the study.

In summary, huPTA-4621 was well-tolerated and significantly decreased the tumor burden in this human HNSCC xenograft model.

## EXAMPLE 4

### *In vivo* Experiment with human Detroit 562 Cells

To demonstrate efficacy against a human HNSCC cancer cell line *in vivo,* huPTA-4621 was tested in a Detroit 562 HNSCC xenograft model. With reference to Figures 6 and 7, 6 to 8 week old female SCID mice were implanted with 3 million Detroit 562 cells in 100 microliters PBS solution injected subcutaneously in the right flank. The mice were randomly divided into 2 treatment groups of 10. When the average tumor volume of the mice reached approximately 188-192mm$^3$, 30 mg/kg of huPTA-4621 test antibody or buffer control was administered intraperitoneally to each cohort in a volume of 100 microliters after dilution from the stock concentration with a diluent that contained 20mM Histidine HCl, 150mM NaCl and 0.01% polysorbate 80, Ph 6.0. The antibody and control samples were then administered once per week for the duration of the study. Tumor growth was measured about every 7 day with calipers. The study was completed after 5 doses of antibody. Body weights of the animals were recorded once per week for the duration of the study. At the end of the study all animals were euthanized according to CCAC guidelines.

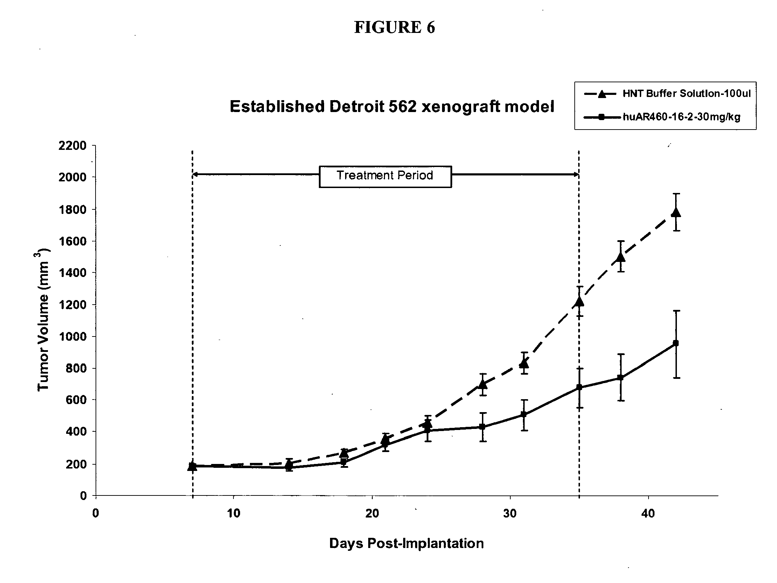

huPTA-4621 reduced tumor growth in the Detroit 562 *in vivo* established model of human HNSCC. Treatment with antibody huPTA-4621 reduced the growth of T.Tn tumors by 52.1 percent (p=0.0259, t-test) compared to the buffer treated group, as determined on day 42, the last day of the study period (Figure 6).

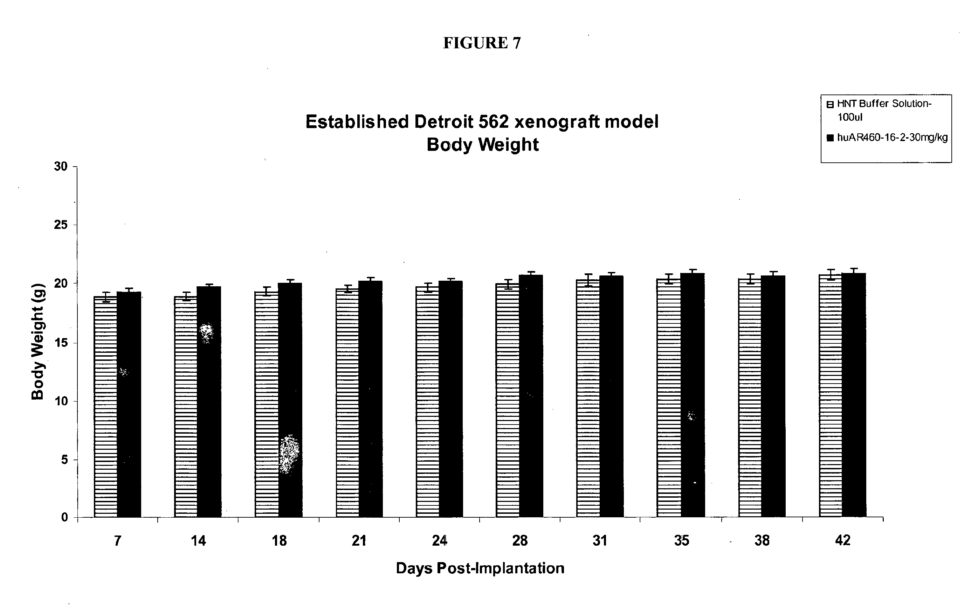

There were no clinical signs of toxicity throughout the study. Body weight measured at weekly intervals was a surrogate for well-being and failure to thrive (Figure 7). There was no significant difference in mean body weight between the groups at the end of the treatment period. There was also no significant difference in mean body weight within each group from the start to the end of the study.

In summary, huPTA-4621 was well-tolerated and significantly decreased the tumor burden in this human HNSCC xenograft model.

## EXAMPLE 5

### *In vivo* Experiment with human CAL 27 Cells

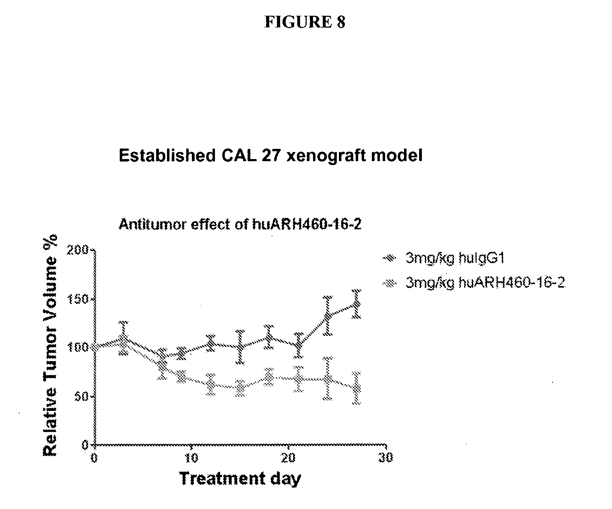

To demonstrate efficacy against a human HNSCC cancer cell line *in vivo*, huPTA-4621 was tested in a CAL 27 HNSCC xenograft model comprising nude mice (NU/J, stock #: 002019, Jackson Laboratories) implanted with the CAL27 cells (ATCC CRL-2095). CAL 27 cells were cultured in DMEM (ATCC, Catalog No. 30-2002) supplemented with 10% FBS (ATCC Catalog No. 30-2020) in 75 mm$^2$ flasks at 37°C in a 5% $CO_2$ in air atmosphere until cells were confluent. Human CAL 27 cells were detached from the culture flasks using 0.25% (w/v) trypsin (Invitrogen, Catalog No. 25200) counted, dissolved in PBS and injected subcutaneously in the mid-back region of 7 week-old male nude mice at a dose of 3x10$^6$ cells/animal with Matrigel™ (BD Biosciences, Catalog No. 354248) in 0.2ml volume (100$\mu$l cells in PBS + 100$\mu$l Matrigel). Following injection of the cells, a period of two weeks elapsed to allow formation of tumor nodules. Tumor volumes (mm$^3$) were measured with a digital caliper (VWR, Catalog No. 36934-152) and estimated by the ellipsoid formula: $[\pi/6]$xaxb$^2$, where a and b are first and second largest orthogonal measurements of the tumor in mm. When tumor volume reached 400-600 mm$^3$, animals were randomized into two groups (n=5). Monoclonal antibody huPTA-4621 and human IgG1 (Sigma, 15154) were administered at 3mg/kg by intraperitoneal injection 3 times per week for a total of 10 doses. Tumor nodules were monitored three times per week using a digital caliper. The antitumor activity was determined in terms of primary tumor growth inhibition by calculating the Relative Tumor Volume (RTV) according to the following formula: $RTV=TV_n/TV_0$, where $TV_n$ is the tumor volume at day n and $TV_0$ is the tumor volume at day 0. Tumor size was followed to day 27. After 27 days of therapy the T/C% was determined by calculating RTV as T/C%=100x (mean RTV of treated group)/(mean RTV of control group). A 60% tumor growth inhibition was found in the antibody treated group (Figure 8), indicating that the treatment was effective in relieving the tumor burden (P<0.0002).

These data clearly demonstrate that huPTA-4621 has significant therapeutic activity against CAL 27 xenograft tumors tested in this study.

## EXAMPLE 6

### *In vivo* Experiment with human CAL 27 Cells

To compare the efficacy of PTA-4621 antibody therapy with standard chemotherapeutic treatment, the model system described in Example 5 was used to compare PTA-4621 therapy with treatment with cisplatin. CAL 27 cells were cultured and harvested as described in Example 5. The CAL 27 xenograft model was established by subcutaneously injecting 7 week old male nude mice (NU/J, stock #: 002019, Jackson Laboratories) in the mid-back region with 5 X 10$^6$ CAL 27 cells in 100 $\mu$l D-PBS with 100 $\mu$l Matrigel. At 18 days after inoculation, the mice were assessed for tumor formation and randomized into 3 groups (for each cohort, n=6): treatment with huPTA-4621, treatment with cisplatin or control (treatment with irrelevant control IgG1 antibody). Tumor volumes ($mm^3$) were measured with a digital caliper (VWR, Catalog No. 36934-152) and estimated by the ellipsoid formula: $[\pi/6]xaxb^2$, where a and b are first and second largest orthogonal measurements of the tumor in mm. At randomization, the average tumor volume was 84 $mm^3$ (range 79-94 $mm^3$).

At randomization animals were i.p. administered test or control antibody at a dose of 3 mg/kg, or cisplatin at a dose of 0.75 mg/kg. Administration volume for all treatments was 6.6 ml/kg. The antibody and control treatments were thereafter administered 3 times per week for the first and third weeks, and twice per week for the second week, resulting in a total of eight treatments. One week after the last treatment the animals were euthanized according to CCAC guidelines; the study was completed on day 46. Tumor growth and body weight were measured three times per week throughout the course of the study.

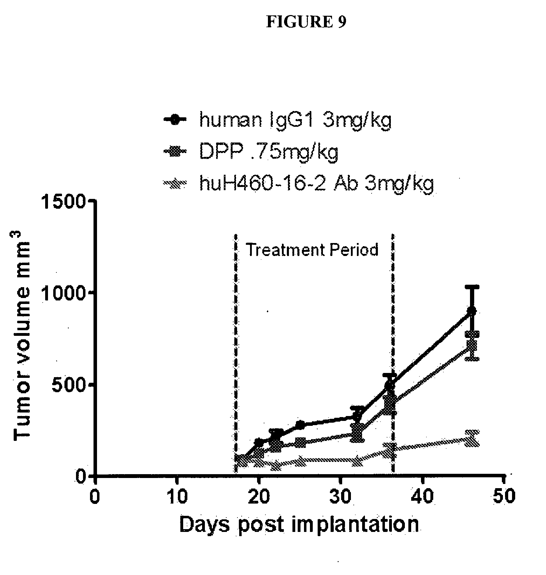

The antitumor activity was determined in terms of primary tumor growth inhibition by calculating the Relative Tumor Volume (RTV) according to the following formula: $RTV=TV_n/TV_0$, where $TV_n$ is the tumor volume at day n and $TV_0$ is the tumor volume at day 0. Tumor size was followed to day 46. At the completion of the study, the T/C% was determined by calculating RTV as T/C%=100x (mean RTV of treated group)/(mean RTV of control group). An 86% tumor growth inhibition was found in the antibody treated group (Figure 9), indicating that the treatment was effective in relieving the tumor burden (p=0.024). No significant growth inhibition was observed in the cisplatin treated group compared to control (Figure 9). Accordingly, huPTA-4621 reduced tumor growth in the CAL 27 *in vivo* established model of human HNSCC.

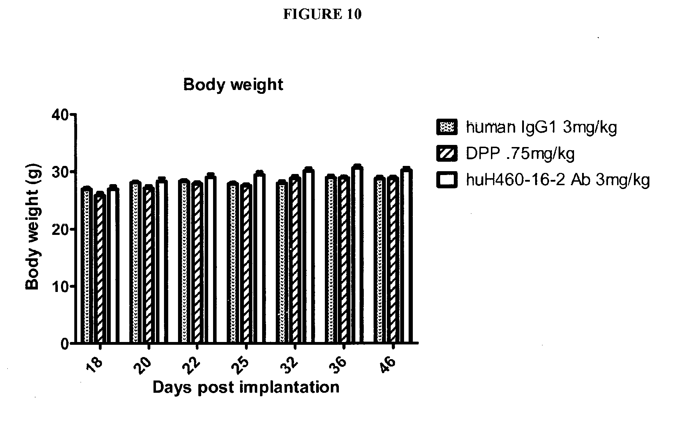

There was no significant difference in mean body weight between the groups at the end of the treatment period. There was also no significant difference in mean body weight within each group from the start to the end of the study (Figure 10). One animal in the control IgG1 group was sacrificed on day 26 because it had reached IACUC endpoints for study determination.

In summary, huPTA-4621 was well-tolerated and significantly decreased the tumor burden relative to standard chemotherapeutics (cisplatin) in this human HNSCC xenograft model.

**Claims**

1.  Use of a monoclonal anti-CD44 antibody, or antigen binding fragment thereof, for the manufacture of a medicament for the treatment of a head and neck squamous cell carcinoma (HNSCC) in a mammal, wherein said HNSCC is **characterized by** the expression of CD44, wherein said antibody comprises

    (a) a $V_H$ CDR1 having the amino acid sequence of SEQ ID NO:3, a $V_H$ CDR2 having the amino acid sequence of SEQ ID NO:4, a $V_H$ CDR3 having the amino acid sequence of SEQ ID NO:5, a $V_L$ CDR1 having the amino acid sequence of SEQ ID NO:6, a $V_L$ CDR2 having the amino acid sequence of SEQ ID NO:7 and a $V_L$ CDR3 having the amino acid sequence of SEQ ID NO:8;
    or
    (b) a $V_H$ domain having an amino acid sequence that is at least 85% identical to the $V_H$ domain having the amino acid sequence of SEQ ID NO:9 or SEQ NO:10 and a $V_L$ domain having an amino acid sequence that is at least 85% identical to the $V_L$ domain having the amino acid sequence of SEQ ID NO:11;
    wherein said antibody competes for binding to CD44 with the antibody produced by the hybridoma deposited with the ATCC with Accession number PTA-4621.

2.  A monoclonal anti-CD44 antibody, or antigen binding fragment thereof, for use in the treatment of a head and neck squamous cell carcinoma (HNSCC) in a mammal, wherein said HNSCC is **characterized by** the expression of CD44, wherein said antibody comprises

(a) a $V_H$ CDR1 having the amino acid sequence of SEQ ID NO:3, a $V_H$ CDR2 having the amino acid sequence of SEQ ID NO:4, a $V_H$ CDR3 having the amino acid sequence of SEQ ID NO:5, a $V_L$ CDR1 having the amino acid sequence of SEQ ID NO:6, a $V_L$ CDR2 having the amino acid sequence of SEQ ID NO:7 and a $V_L$ CDR3 having the amino acid sequence of SEQ ID NO:8;

or

(b) a $V_H$ domain having an amino acid sequence that is at least 85% identical to the $V_H$ domain having the amino acid sequence of SEQ ID NO:9 or SEQ NO:10 and a $V_L$ domain having an amino acid sequence that is at least 85% identical to the $V_L$ domain having the amino acid sequence of SEQ ID NO: 11;

wherein said antibody competes for binding to CD44 with the antibody produced by the hybridoma deposited with the ATCC with Accession number PTA-4621.

3. The use of claim 1 or the antibody or fragment for use according to claim 2, wherein said anti-CD44 antibody is a chimeric version of the monoclonal antibody produced by the hybridoma deposited with the ATCC with accession number PTA-4621.

4. The use of claim 1 or the antibody or fragment for use according to claim 2, wherein said anti-CD44 antibody is a humanized version of the monoclonal antibody produced by the hybridoma deposited with the ATCC with accession number PTA-4621.

5. The use of claim 1 or 3, or the antibody or fragment for use according to claim 2 or 3, wherein said anti-CD44 antibody comprises a $V_H$ domain having the amino acid sequence of SEQ ID NO:1.

6. The use of claim 1, 3 or 5, or the antibody or fragment for use according to claim 2, 3 or 5, wherein said anti-CD44 antibody comprises a $V_L$ domain having the amino acid sequence of SEQ ID NO:2.

7. The use of claim 1 or 4, or the antibody or fragment for use according to claim 1 or 4, wherein said anti-CD44 antibody comprises a $V_H$ domain having the amino acid sequence of SEQ ID NO:9 or SEQ ID NO: 10.

8. The use of claim 1, 4 or 7, or the antibody or fragment for use according to claim 1, 4 or 7, wherein said anti-CD44 antibody comprises a $V_L$ domain having the amino acid sequence of SEQ ID NO:11.

9. The use of any one of claims 1 and 3-8, or the antibody or fragment for use according to any one of claims 2-8, wherein said antibody or fragment is conjugated to a therapeutic or reporter moiety or to hematogenous cells.

10. Use of a humanized anti-CD44 antibody, or antigen binding fragment thereof, for the manufacture of a medicament for the treatment of a head and neck squamous cell carcinoma (HNSCC) in a human, wherein said HNSCC is **characterized by** the expression of CD44 and wherein said humanized antibody comprises a $V_H$ domain having the amino acid sequence of SEQ ID NO:9 and a $V_L$ domain having the amino acid sequence of SEQ ID NO: 11, or comprises a $V_H$ domain having the amino acid sequence of SEQ ID NO: 10 and a $V_L$ domain having the amino acid sequence of SEQ ID NO:11.

11. A humanized anti-CD44 antibody, or antigen binding fragment thereof, for use in the treatment of a head and neck squamous cell carcinoma (HNSCC) in a human, wherein said HNSCC is **characterized by** the expression of CD44 and wherein said humanized antibody comprises a $V_H$ domain having the amino acid sequence of SEQ ID NO:9 and a $V_L$ domain having the amino acid sequence of SEQ ID NO: 11, or comprises a $V_H$ domain having the amino acid sequence of SEQ ID NO:10 and a $V_L$ domain having the amino acid sequence of SEQ ID NO: 11.

**Patentansprüche**

1. Verwendung eines monoclonalen Anti-CD44-Antikörpers oder eines Antigen-bindenden Fragments davon, zur Herstellung eines Arzneimittels für die Behandlung eines Kopf-Hals-Plattenepithelkarzinoms (HNSCC) bei einem Säuger, wobei das HNSCC durch die Expression von CD44 gekennzeichnet ist, wobei der Antikörper umfasst:

(a) eine $V_H$-CDR1 mit der Aminosäuresequenz von SEQ ID NO:3, eine $V_H$-CDR2 mit der Aminosäuresequenz von SEQ ID NO:4, eine $V_H$-CDR3 mit der Aminosäuresequenz von SEQ ID NO:5, eine $V_L$-CDR1 mit der Aminosäuresequenz von SEQ ID NO:6, eine $V_L$-CDR2 mit der Aminosäuresequenz von SEQ ID NO:7 und eine $V_L$-CDR3 mit der Aminosäuresequenz von SEQ ID NO:8;

oder

(b) eine $V_H$-Domäne mit einer Aminosäuresequenz, die zu mindestens 85% identisch ist mit der $V_H$-Domäne, die die Aminosäuresequenz von SEQ ID NO:9 oder SEQ ID NO:10 hat und eine $V_L$-Domäne mit einer Aminosäuresequenz, die zu mindestens 85% identisch ist mit der $V_L$-Domäne, die die Aminosäuresequenz von SEQ ID NO:11 hat;

wobei der Antikörper um die Bindung an CD44 mit dem Antikörper konkurriert, der durch das Hybridom hergestellt wird, das unter der ATCC-Hinterlegungsnummer PTA-4621 hinterlegt ist.

2. Monoclonaler Anti-CD44-Antikörper oder Antigen-bindendes Fragment davon, zur Verwendung bei der Behandlung eines Kopf-Hals-Plattenepithelkarzinoms (HNSCC) bei einem Säuger, wobei das HNSCC durch die Expression von CD44 gekennzeichnet ist, wobei der Antikörper umfasst:

(a) eine $V_H$-CDR1 mit der Aminosäuresequenz von SEQ ID NO:3, eine $V_H$-CDR2 mit der Aminosäuresequenz von SEQ ID NO:4, eine $V_H$-CDR3 mit der Aminosäuresequenz von SEQ ID NO:5, eine $V_L$-CDR1 mit der Aminosäuresequenz von SEQ ID NO:6, eine $V_L$-CDR2 mit der Aminosäuresequenz von SEQ ID NO:7 und eine $V_L$-CDR3 mit der Aminosäuresequenz von SEQ ID NO:8;
oder

(b) eine $V_H$-Domäne mit einer Aminosäuresequenz, die zu mindestens 85% identisch ist mit der $V_H$-Domäne, die die Aminosäuresequenz von SEQ ID NO:9 oder SEQ ID NO:10 aufweist und eine $V_L$-Domäne mit einer Aminosäuresequenz, die zu mindestens 85% identisch ist mit der $V_L$-Domäne, die die Aminosäuresequenz von SEQ ID NO:11 aufweist;

wobei der Antikörper um die Bindung an CD44 mit dem Antikörper konkurriert, der durch das Hybridom hergestellt wird, das unter der ATCC-Hinterlegungsnummer PTA-4621 hinterlegt ist.

3. Verwendung nach Anspruch 1 oder Antikörper oder Fragment zur Verwendung nach Anspruch 2, wobei der Anti-CD44-Antikörper eine chimäre Version des monoclonalen Antikörpers ist, der durch das Hybridom hergestellt wird, das unter der ATCC-Hinterlegungsnummer PTA-4621 hinterlegt ist.

4. Verwendung nach Anspruch 1 oder Antikörper oder Fragment zur Verwendung nach Anspruch 2, wobei der Anti-CD44-Antikörper eine humanisierte Version des monoclonalen Antikörpers ist, der durch das Hybridom hergestellt wird, das unter der ATCC-Hinterlegungsnummer PTA-4621 hinterlegt ist.

5. Verwendung nach Anspruch 1 oder 3, oder Antikörper oder Fragment zur Verwendung nach Anspruch 2 oder 3, wobei der Anti-CD44-Antikörper eine $V_H$-Domäne mit der Aminosäuresequenz von SEQ ID NO:1 umfasst.

6. Verwendung nach Anspruch 1, 3 oder 5, oder Antikörper oder Fragment zur Verwendung nach Anspruch 2, 3 oder 5, wobei der Anti-CD44-Antikörper eine $V_L$-Domäne mit der Aminosäuresequenz von SEQ ID NO:2 umfasst.

7. Verwendung nach Anspruch 1 oder 4, oder Antikörper oder Fragment zur Verwendung nach Anspruch 1 oder 4, wobei der Anti-CD44-Antikörper eine $V_H$-Domäne mit der Aminosäuresequenz von SEQ ID NO:9 oder SEQ ID NO: 10 umfasst.

8. Verwendung nach Anspruch 1, 4 oder 7, oder Antikörper oder Fragment zur Verwendung nach Anspruch 1, 4 oder 7, wobei der Anti-CD44-Antikörper eine $V_L$-Domäne mit der Aminosäuresequenz von SEQ ID NO:11 umfasst.

9. Verwendung nach einem der Ansprüche 1 und 3 bis 8, oder Antikörper oder Fragment zur Verwendung nach einem der Ansprüche 2 bis 8, wobei der Antikörper oder das Fragment mit einer therapeutischen funktionellen Gruppe oder einer funktionellen Reporter-Gruppe oder mit hämatogenen Zellen verbunden ist.

10. Verwendung eines humanisierten Anti-CD44-Antikörpers oder eines Antigen-bindenden Fragments davon, zur Herstellung eines Arzneimittels für die Behandlung eines Kopf-Hals-Plattenepithelkarzinoms (HNSCC) bei einem Menschen, wobei das HNSCC durch die Expression von CD44 gekennzeichnet ist und wobei der humanisierte Antikörper eine $V_H$-Domäne mit der Aminosäuresequenz von SEQ ID NO:9 und eine $V_L$-Domäne mit der Aminosäuresequenz von SEQ ID NO:11 umfasst, oder eine $V_H$-Domäne mit der Aminosäuresequenz von SEQ ID NO:10 und eine $V_L$-Domäne mit der Aminosäuresequenz von SEQ ID NO:11 umfasst.

11. Humanisierter Anti-CD44-Antikörper oder Antigen-bindendes Fragment davon, zur Verwendung bei der Behandlung eines Kopf-Hals-Plattenepithelkarzinoms (HNSCC) in einem Menschen, wobei das HNSCC durch die Expression von CD44 gekennzeichnet ist und wobei der humanisierte Antikörper eine $V_H$-Domäne mit der Aminosäuresequenz von SEQ ID NO:9 und eine $V_L$-Domäne mit der Aminosäuresequenz von SEQ ID NO:11 umfasst, oder eine $V_H$-Domäne mit der Aminosäuresequenz von SEQ ID NO:10 und eine $V_L$-Domäne mit der Aminosäuresequenz von SEQ ID NO:11 umfasst.

**Revendications**

1. Utilisation d'un anticorps monoclonal anti-CD44, ou d'un fragment de celui-ci se liant à un antigène, pour la fabrication d'un médicament destiné au traitement d'un carcinome épidermoïde de la tête et du cou (HNSCC) chez un mammifère, où ledit HNSCC est **caractérisé par** l'expression de CD44, où ledit anticorps comprend

   (a) une CDR1 de $V_H$ ayant la séquence d'acides aminés de SEQ ID NO: 3, une CDR2 de $V_H$ ayant la séquence d'acides aminés de SEQ ID NO: 4, une CDR3 de $V_H$ ayant la séquence d'acides aminés de SEQ ID NO: 5, une CDR1 de $V_L$ ayant la séquence d'acides aminés de SEQ ID NO: 6, une CDR2 de $V_L$ ayant la séquence d'acides aminés de SEQ ID NO: 7 et une CDR3 de $V_L$ ayant la séquence d'acides aminés de SEQ ID NO: 8 ; ou
   (b) un domaine $V_H$ ayant une séquence d'acides aminés qui est au moins à 85 % identique au domaine $V_H$ ayant la séquence d'acides aminés de SEQ ID NO: 9 ou SEQ ID NO: 10 et un domaine $V_L$ ayant une séquence d'acides aminés qui est au moins à 85 % identique au domaine $V_L$ ayant la séquence d'acides aminés de SEQ ID NO: 11 ;
   où ledit anticorps entre en compétition pour une liaison à CD44 avec l'anticorps produit par l'hybridome déposé auprès de l'ATCC sous le numéro d'accès PTA-4621.

2. Anticorps monoclonal anti-CD44, ou fragment de celui-ci se liant à un antigène, destiné à être utilisé dans le traitement d'un carcinome épidermoïde de la tête et du cou (HNSCC) chez un mammifère, où ledit HNSCC est **caractérisé par** l'expression de CD44, où ledit anticorps comprend

   (a) une CDR1 de $V_H$ ayant la séquence d'acides aminés de SEQ ID NO: 3, une CDR2 de $V_H$ ayant la séquence d'acides aminés de SEQ ID NO: 4, une CDR3 de $V_H$ ayant la séquence d'acides aminés de SEQ ID NO: 5, une CDR1 de $V_L$ ayant la séquence d'acides aminés de SEQ ID NO: 6, une CDR2 de $V_L$ ayant la séquence d'acides aminés de SEQ ID NO: 7 et une CDR3 de $V_L$ ayant la séquence d'acides aminés de SEQ ID NO: 8 ; ou
   (b) un domaine $V_H$ ayant une séquence d'acides aminés qui est au moins à 85 % identique au domaine $V_H$ ayant la séquence d'acides aminés de SEQ ID NO: 9 ou SEQ ID NO: 10 et un domaine $V_L$ ayant une séquence d'acides aminés qui est au moins à 85 % identique au domaine $V_L$ ayant la séquence d'acides aminés de SEQ ID NO: 11 ;
   où ledit anticorps entre en compétition pour une liaison à CD44 avec l'anticorps produit par l'hybridome déposé auprès de l'ATCC sous le numéro d'accès PTA-4621.

3. Utilisation selon la revendication 1 ou anticorps ou fragment destiné à être utilisé selon la revendication 2, où ledit anticorps anti-CD44 est une version chimérique de l'anticorps monoclonal produit par l'hybridome déposé auprès de l'ATCC sous le numéro d'accès PTA-4621.

4. Utilisation selon la revendication 1 ou anticorps ou fragment destiné à être utilisé selon la revendication 2, où ledit anticorps anti-CD44 est une version humanisée de l'anticorps monoclonal produit par l'hybridome déposé auprès de l'ATCC sous le numéro d'accès PTA-4621.

5. Utilisation selon la revendication 1 ou 3, ou anticorps ou fragment destiné à être utilisé selon la revendication 2 ou 3, où ledit anticorps anti-CD44 comprend un domaine $V_H$ ayant la séquence d'acides aminés de SEQ ID NO: 1.

6. Utilisation selon la revendication 1, 3 ou 5, ou anticorps ou fragment destiné à être utilisé selon la revendication 2, 3 ou 5, où ledit anticorps anti-CD44 comprend un domaine $V_L$ ayant la séquence d'acides aminés de SEQ ID NO: 2.

7. Utilisation selon la revendication 1 ou 4, ou anticorps ou fragment destiné à être utilisé selon la revendication 1 ou 4, où ledit anticorps anti-CD44 comprend un domaine $V_H$ ayant la séquence d'acides aminés de SEQ ID NO: 9 ou SEQ ID NO: 10.

8. Utilisation selon la revendication 1, 4 ou 7, ou anticorps ou fragment destiné à être utilisé selon la revendication 1, 4 ou 7, où ledit anticorps anti-CD44 comprend un domaine $V_L$ ayant la séquence d'acides aminés de SEQ ID NO: 11.

9. Utilisation selon l'une quelconque des revendications 1 et 3 à 8, ou anticorps ou fragment destiné à être utilisé selon l'une quelconque des revendications 2 à 8, où ledit anticorps ou fragment est conjugué à un fragment thérapeutique ou rapporteur ou à des cellules hématogènes.

10. Utilisation d'un anticorps anti-CD44 humanisé, ou d'un fragment de celui-ci se liant à un antigène, pour la fabrication d'un médicament destiné au traitement d'un carcinome épidermoïde de la tête et du cou (HNSCC) chez un humain, où ledit HNSCC est **caractérisé par** l'expression de CD44 et où ledit anticorps humanisé comprend un domaine $V_H$ ayant la séquence d'acides aminés de SEQ ID NO: 9 et un domaine $V_L$ ayant la séquence d'acides aminés de SEQ ID NO: 11, ou comprend un domaine $V_H$ ayant la séquence d'acides aminés de SEQ ID NO: 10 et un domaine $V_L$ ayant la séquence d'acides aminés de SEQ ID NO: 11.

11. Anticorps anti-CD44 humanisé, ou fragment de celui-ci se liant à un antigène, destiné à être utilisé dans le traitement d'un carcinome épidermoïde de la tête et du cou (HNSCC) chez un humain, où ledit HNSCC est **caractérisé par** l'expression de CD44 et où ledit anticorps humanisé comprend un domaine $V_H$ ayant la séquence d'acides aminés de SEQ ID NO: 9 et un domaine $V_L$ ayant la séquence d'acides aminés de SEQ ID NO: 11, ou comprend un domaine $V_H$ ayant la séquence d'acides aminés de SEQ ID NO: 10 et un domaine $V_L$ ayant la séquence d'acides aminés de SEQ ID NO: 11.

# FIGURE 1A

# FIGURE 1B

| | Sample Name | Mean, APC-A |
|---|---|---|
| — · — · — | CAL 27 #1 Rituximab.fcs | 965.14 |
| —————— | CAL 27 #1 huCD44.fcs | 35283.00 |
| ————— | CAL 27 #2 Rituximab.fcs | 886.42 |
| - - - - - - | CAL 27 #2 huCD44.fcs | 33030.21 |

FIGURE 2

Established T.Tn xenograft model

EP 2 531 527 B1

FIGURE 3

Established T.Tn xenograft model
Body Weight

EP 2 531 527 B1

FIGURE 4

Established SCC-15 xenograft model

HNT Buffer-100 ul
huARH460-16-2-30 mg/kg

TGI=45%, p=0.0379

Treatment Period

Tumor Volume (mm³)

Days Post-Implantation

FIGURE 5

Established SCC-15 xenograft model
Body Weight

☐ HNT Buffer-100 ul

■ huARH460-16-2-30 mg/kg

EP 2 531 527 B1

FIGURE 6

Established Detroit 562 xenograft model

EP 2 531 527 B1

FIGURE 7

## Established Detroit 562 xenograft model
## Body Weight

**Days Post-Implantation**

Legend:
- ⊟ HNT Buffer Solution-100ul
- ■ huAR460-16-2-30mg/kg

FIGURE 8

**Established CAL 27 xenograft model**

Antitumor effect of huARH460-16-2

**FIGURE 9**

FIGURE 10

# FIGURE 11

```
                                                                    ---CDR1-
-
E  V  K  L  L  E  S  G  G  G  L  V  Q  P  G  G  S  L  K  L  S  C  A  T  S  G  F  D  F  S  R  Y  W
         M
                    10                         20                            30


-                                    -------------------CDR2---------------------------
S  W  V  R  Q  A  P  G  K  G  L  E  W  I  G  E  V  N  P  D  S  T  S  I  N  Y  T  P  S  L  K  D  Q
         F
         40                      50      52 A                    60


                                                                       -------
CD
I  I  S  R  D  N  A  K  N  T  L  D  L  Q  M  S  K  V  S  S  E  D  T  A  L  Y  Y  C  T  R  P  N  Y
Y
   70             .               80      82 A  B  C                  90


R3-------------------------
G  S  R  Y  H  Y  Y  A  M  D  Y  W  G  Q  G  T  S  V  T  V  S  S
100 A  B  C  D  E  F  G                      110
```

**FIGURE 12**

```
                                                      --------------CDR1----------
-
D   I   Q   M   T   Q   T   T   S   S   L   S   V   S   L   G   D   R   V   T   I   N   C   R   A   S   Q   D   I   N   N   Y   L
            N
                            10                                      20                                      30

                                                  ------CDR2-------
W   Y   Q   Q   K   P   D   G   T   V   K   L   L   I   Y   Y   T   S   R   L   H   S   G   V   P   S   R   F   S   G   S   G   S
G
                    40                                      50                                      60

                                                  ---------CDR3---------
T   D   F   S   L   T   I   S   N   L   E   K   E   D   V   A   T   Y   F   C   Q   Q   G   S   T   L   P   F   T   F   G   S   G
T
    70                                      80                                      90                                      100

K   L   E   I   K
        106 A
```

## FIGURE 13

Heavy Chain HV1:

```
                                                                    ----CDR1--
E   V   Q   L   V   E   S   G   G   G   L   V   Q   P   G   G   S   L   R   L   S   C   A   A   S   G   F   D   F   S   R   Y   W   M
                    10                      20                          30
```

```
                                            -----------------------CDR2----------------------
S   W   V   R   Q   A   P   G   K   G   L   V   W   V   G   E   V   N   P   D   S   T   S   I   N   Y   T   P   S   L   K   D   R   F
                    40                          50      52  A                          60
```

```
                                                                            -------CD-
T   I   S   R   D   N   A   K   N   T   L   Y   L   Q   M   N   S   L   R   A   E   D   T   A   V   Y   Y   C   T   R   P   N   Y   Y
        70                              80      82  A   B   C                          90
```

```
R3------------------------------
G   S   R   Y   H   Y   Y   A   M   D   Y   W   G   Q   G   T   L   V   T   V   S   S
    100 A   B   C   D   E   F   G                       110
```


Heavy Chain HV2:

```
                                                                    -----CDR1--
E   V   Q   L   V   E   S   G   G   G   L   V   Q   P   G   G   S   L   R   L   S   C   A   T   S   G   F   D   F   S   R   Y   W   M
                    10                      20                          30
```

```
                                            -----------------------CDR2----------------------
S   W   V   R   Q   A   P   G   K   G   L   V   W   I   G   E   V   N   P   D   S   T   S   I   N   Y   T   P   S   L   K   D   Q   F
                    40                          50      52  A                          60
```

```
                                                                            --------CD
T   I   S   R   D   N   A   K   N   T   L   Y   L   Q   M   N   S   L   R   A   E   D   T   A   V   Y   Y   C   T   R   P   N   Y   Y
        70                              80      82  A   B   C                          90
```

```
R3------------------------------
G   S   R   Y   H   Y   Y   A   M   D   Y   W   G   Q   G   T   L   V   T   V   S   S
    100 A   B   C   D   E   F   G                       110
```

EP 2 531 527 B1

EP 2 531 527 B1

## FIGURE 14

Light Chain KV1:

```
                                                       ------------------CDR1------
D   I   Q   M   T   Q   S   P   S   S   L   S   A   S   V   G   D   R   V   T   I   T   C   R   A   S   Q   D   I   N   N   Y
                        10                      20                          30
```

```
----
L   N   W   Y   Q   Q   K   P   G   K   A   P   K   L   L   I   Y   Y   T   S   R   L   H   S   G   V   P   S   R   F   S   G   S
                        40                                          50                              60
```

```
                                                      ----------CDR3------------
G   S   G   T   D   F   T   F   T   I   S   S   L   Q   P   E   D   I   A   T   Y   Y   C   Q   Q   G   S   T   L   P   F   T   F
                70                              80                              90
```

```
G   Q   G   T   K   L   E   I   K
    100                 106 A
```

## REFERENCES CITED IN THE DESCRIPTION

### Patent documents cited in the description

- WO 02094879 A
- US 20030103985 A
- US 2009004103 A
- US 6180357 B
- US 7252821 B
- US 8048416 B
- US 7507537 B
- US 8071072 B
- US 4816567 A
- US 61301449 B

### Non-patent literature cited in the description

- **DA CRUZ.** *European J. of Cancer,* 2008, vol. 6, 170
- **KABAT et al.** Sequences of Proteins of Immunological Interest. Public Health Service, National Institutes of Health, 1991
- **CHOTHIA ; LESK.** *J. Mol. Biol.,* 1987, vol. 196, 901-917
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **SAMBROOK ; RUSSELL.** Molecular Cloning, A Laboratory Manual. Cold Spring Harbor Laboratory, 2001
- **AUSUBEL.** Current Protocols in Molecular Biology. Green Publishing Associates and Wiley Interscience, 1989
- Nucleic acid hybridization, a practical approach. IRL Press, 1985
- **THOMPSON.** *Nucl. Acids Res.,* 1994, vol. 2, 4673-4680
- **BRUTLAG.** *Comp. App. Biosci.,* 1990, vol. 6, 237-245
- **ALTSCHUL.** *Nucl. Acids Res.,* 1997, vol. 25, 3389-3402
- **ALTSCHUL.** *J. Mol. Evol.,* 1993, vol. 36, 290-300
- **ALTSCHUL.** *J. Mol. Biol.,* 1990, vol. 215, 403-410
- **HENIKOFF.** *PNAS,* 1989, vol. 89, 10915
- **PEDLEY et al.** *Br. J. Cancer,* 1993, vol. 68, 69-73
- **THERASSE et al.** New guidelines to evaluate the response to treatment in solid tumors. European Organization for Research and Treatment of Cancer, National Cancer Institute of the United States, National Cancer Institute of Canada. *J Natl Cancer Inst.,* 2000, vol. 92, 205-16

SEQUENCE LISTING

<110> F. Hoffmann-La Roche AG
The University of Miami - Dept. of Medicine

<120> METHODS FOR THE TREATMENT OF HEAD AND NECK SQUAMOUS CELL CARCINOMA

<130> 26583 WO

<150> US 61/301,449
<151> 2010-02-04

<160> 12

<170> Patent In version 3.2

<210> 1
<211> 124
<212> PRT
<213> Homo sapiens

<400> 1

```
Glu Val Lys Leu Leu Glu Ser Gly Gly Gly Leu Val Gln Pro Gly Gly
1               5               10              15

Ser Leu Lys Leu Ser Cys Ala Thr Ser Gly Phe Asp Phe Ser Arg Tyr
            20              25              30

Trp Met Ser Trp Val Arg Gln Ala Pro Gly Lys Gly Leu Glu Trp Ile
            35              40              45

Gly Glu Val Asn Pro Asp Ser Thr Ser Ile Asn Tyr Thr Pro Ser Leu
    50              55              60

Lys Asp Gln Phe Ile Ile Ser Arg Asp Asn Ala Lys Asn Thr Leu Asp
65              70              75              80

Leu Gln Met Ser Lys Val Ser Ser Glu Asp Thr Ala Leu Tyr Tyr Cys
            85              90              95

Thr Arg Pro Asn Tyr Tyr Gly Ser Arg Tyr His Tyr Tyr Ala Met Asp
            100             105             110

Tyr Trp Gly Gln Gly Thr Ser Val Thr Val Ser Ser
        115             120
```

<210> 2
<211> 107
<212> PRT
<213> Homo sapiens

<400> 2

```
Asp Ile Gln Met Thr Gln Thr Thr Ser Ser Leu Ser Val Ser Leu Gly
1               5                   10                  15

Asp Arg Val Thr Ile Asn Cys Arg Ala Ser Gln Asp Ile Asn Asn Tyr
            20                  25                  30

Leu Asn Trp Tyr Gln Gln Lys Pro Asp Gly Thr Val Lys Leu Leu Ile
            35                  40                  45

Tyr Tyr Thr Ser Arg Leu His Ser Gly Val Pro Ser Arg Phe Ser Gly
    50                  55                  60

Ser Gly Ser Gly Thr Asp Phe Ser Leu Thr Ile Ser Asn Leu Glu Lys
65                  70                  75                  80

Glu Asp Val Ala Thr Tyr Phe Cys Gln Gln Gly Ser Thr Leu Pro Phe
                85                  90                  95

Thr Phe Gly Ser Gly Thr Lys Leu Glu Ile Lys
            100                 105
```

<210> 3
<211> 5
<212> PRT
<213> Homo sapiens

<400> 3

```
                        Arg Tyr Trp Met Ser
                        1               5
```

<210> 4
<211> 17
<212> PRT
<213> Homo sapiens

<400> 4

```
Glu Val Asn Pro Asp Ser Thr Ser Ile Asn Tyr Thr Pro Ser Leu Lys
1               5                   10                  15

Asp
```

<210> 5
<211> 15
<212> PRT
<213> Homo sapiens

<400> 5

Pro Asn Tyr Tyr Gly Ser Arg Tyr His Tyr Tyr Ala Met Asp Tyr
1                   5                   10                  15

<210> 6
<211> 11
<212> PRT
<213> Homo sapiens

<400> 6

Arg Ala Ser Gln Asp Ile Asn Asn Tyr Leu Asn
1                   5                   10

<210> 7
<211> 7
<212> PRT
<213> Homo sapiens

<400> 7

Tyr Thr Ser Arg Leu His Ser
1                   5

<210> 8
<211> 9
<212> PRT
<213> Homo sapiens

<400> 8

Gln Gln Gly Ser Thr Leu Pro Phe Thr
1                   5

<210> 9
<211> 124
<212> PRT
<213> Homo sapiens

<400> 9

Glu Val Gln Leu Val Glu Ser Gly Gly Gly Leu Val Gln Pro Gly Gly
1                   5                   10                  15

Ser Leu Arg Leu Ser Cys Ala Ala Ser Gly Phe Asp Phe Ser Arg Tyr
            20                  25                  30

Trp Met Ser Trp Val Arg Gln Ala Pro Gly Lys Gly Leu Val Trp Val
                35                  40                  45

Gly Glu Val Asn Pro Asp Ser Thr Ser Ile Asn Tyr Thr Pro Ser Leu
        50                  55                  60

Lys Asp Arg Phe Thr Ile Ser Arg Asp Asn Ala Lys Asn Thr Leu Tyr
65                70                75                80

Leu Gln Met Asn Ser Leu Arg Ala Glu Asp Thr Ala Val Tyr Tyr Cys
                85                90                95

Thr Arg Pro Asn Tyr Tyr Gly Ser Arg Tyr His Tyr Tyr Ala Met Asp
            100                105                110

Tyr Trp Gly Gln Gly Thr Leu Val Thr Val Ser Ser
        115                120

<210> 10
<211> 124
<212> PRT
<213> Homo sapiens

<400> 10

Glu Val Gln Leu Val Glu Ser Gly Gly Gly Leu Val Gln Pro Gly Gly
1                5                10                15

Ser Leu Arg Leu Ser Cys Ala Thr Ser Gly Phe Asp Phe Ser Arg Tyr
            20                25                30

Trp Met Ser Trp Val Arg Gln Ala Pro Gly Lys Gly Leu Val Trp Ile
            35                40                45

Gly Glu Val Asn Pro Asp Ser Thr Ser Ile Asn Tyr Thr Pro Ser Leu
        50                55                60

Lys Asp Gln Phe Thr Ile Ser Arg Asp Asn Ala Lys Asn Thr Leu Tyr
65                70                75                80

Leu Gln Met Asn Ser Leu Arg Ala Glu Asp Thr Ala Val Tyr Tyr Cys
                85                90                95

Thr Arg Pro Asn Tyr Tyr Gly Ser Arg Tyr His Tyr Tyr Ala Met Asp
            100                105                110

Tyr Trp Gly Gln Gly Thr Leu Val Thr Val Ser Ser
        115                120

<210> 11
<211> 107
<212> PRT
<213> Homo sapiens

<400> 11

```
Asp Ile Gln Met Thr Gln Ser Pro Ser Ser Leu Ser Ala Ser Val Gly
1               5               10              15


Asp Arg Val Thr Ile Thr Cys Arg Ala Ser Gln Asp Ile Asn Asn Tyr
            20              25              30


Leu Asn Trp Tyr Gln Gln Lys Pro Gly Lys Ala Pro Lys Leu Leu Ile
        35              40              45


Tyr Tyr Thr Ser Arg Leu His Ser Gly Val Pro Ser Arg Phe Ser Gly
    50              55              60


Ser Gly Ser Gly Thr Asp Phe Thr Phe Thr Ile Ser Ser Leu Gln Pro
65              70              75              80


Glu Asp Ile Ala Thr Tyr Tyr Cys Gln Gln Gly Ser Thr Leu Pro Phe
            85              90              95


Thr Phe Gly Gln Gly Thr Lys Leu Glu Ile Lys
        100             105
```

<210> 12
<211> 11
<212> PRT
<213> Homo sapiens

<400> 12

```
Ala Phe Asp Gly Pro Ile Thr Ile Thr Ile Val
1               5               10
```